# EUROPEAN PATENT SPECIFICATION

(11) **EP 1 357 163 B1**
(45) Date of publication and mention of the grant of the patent: **31.05.2006**
(21) Application number: 03006887.8
(22) Date of filing: 31.03.2003
(51) Int. Cl.: C09K 19/34, C07D 333/50, C09K 19/38

(54) **Reactive mesogenic benzodithiophenes**
Reaktive mesogene Benzodithiophene
Benzodithiophènes mésogéniques réactifs

(30) Priority: 24.04.2002 EP 02009083
(43) Date of publication of application: 29.10.2003
(73) Proprietor: Merck Patent GmbH, 64293 Darmstadt (DE)
(72) Inventor: Farrand, Louise, Dr., Blandford Forum, DT11 9ED (GB); Heeney, Martin, Southampton SO14 6TQ (GB); Tierny, Steven, Southampton SO15 7QW (GB); Giles, Mark, Southampton SO15 2LE (GB); Thompson, Marcus, Hamshire SP6 1RR (GB); Shkunov, Maxim, Southampton SO16 6SX (GB); Sparrowe, David, Bournemouth, Dorset BH5 5EJ (GB); McCulloch, Iain, Kings Somborne, Hants SO20 6PE (GB)

(56) References cited:
- EP-A- 1 138 713
- EP-A- 1 275 651
- WO-A-93/22397
- WO-A-97/00600
- WO-A-98/04544
- WO-A-99/12989
- WO-A-03/006468
- US-A- 6 150 668
- DATABASE INSPEC [Online] INSTITUTE OF ELECTRICAL ENGINEERS, STEVENAGE, GB; KOBAYASHI K ET AL: "Crystal structures and physico-chemical properties of thiophene-fused TCNQ acceptors and their TTF complexes" Database accession no. 3379704 XP002244917 & INTERNATIONAL CONFERENCE ON SCIENCE AND TECHNOLOGY OF SYNTHETIC METALS (ICSM '88), SANTA FE, NM, USA, 26 JUNE-2 JULY 1988, vol. 27, no. 3-4, pages B309-B314, Synthetic Metals, 30 Dec. 1988, Switzerland ISSN: 0379-6779
- PATENT ABSTRACTS OF JAPAN vol. 012, no. 372 (C-533), 5 October 1988 (1988-10-05) -& JP 63 122727 A (RICOH CO LTD), 26 May 1988 (1988-05-26)

## Description

### Field of Invention

The invention relates to new reactive mesogenic benzodithiophene derivatives. The invention further relates to their use as semiconductors or charge transport materials, in optical, electro-optical or electronic devices like for example liquid crystal displays, optical films, organic field effect transistors (FET or OFET) for thin film transistor liquid crystal displays and integrated circuit devices such as RFID tags, electroluminescent devices in flat panel displays, and in photovoltaic and sensor devices. The invention further relates to a field effect transistor, light emitting device or ID tag comprising the reactive mesogenic benzodithiophenes.

### Background and Prior Art

Organic materials have recently shown promise as the active layer in organic based thin film transistors and organic field effect transistors [see H. E. Katz, Z. Bao and S. L. Gilat, *Acc. Chem. Res*., 2001, **34**, 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semi-conducting material and the current on/off ratio, so the ideal semiconductor should have a low conductivity in the off state, combined with a high charge carrier mobility (> 1 x 10⁻³ cm²V⁻¹ s⁻¹). In addition, it is important that the semi-conducting material is relatively stable to oxidation i.e. it has a high ionisation potential, as oxidation leads to reduced device performance.

A known compound which has been shown to be an effective p-type semiconductor for OFETs is pentacene [see S. F. Nelson, Y. Y. Lin, D. J. Gundlach and T. N. Jackson, *Appl. Phys. Lett.,* 1998, **72,** 1854]. When deposited as a thin film by vacuum deposition, it was shown to have carrier mobilities in excess of 1 cm² V⁻¹ s⁻¹ with very high current on/off ratios greater than 10⁶. However, vacuum deposition is an expensive processing technique that is unsuitable for the fabrication of large-area films.

Regular poly(3-hexylthiophene) has been reported with charge carrier mobility between 1 x 10⁻⁵ and 4.5 x 10⁻² cm² V⁻¹ s⁻¹, but with a rather low current on/off ratio between 10 and 10³ [see Z. Bao et al., *Appl. Phys. Lett.* 1997, **78,** 2184]. In general, poly(3-alkylthiophenes) show improved solubility and are able to be solution processed to fabricate large area films. However, poly(3-alkylthiophenes) have relatively low ionisation potentials and are susceptible to doping in air [see H. Sirringhaus et al., *Adv. Solid State Phys*. 1999, **39**, 101].

It was an aim of the present invention to provide new organic materials for use as semiconductors or charge transport materials, which are easy to synthesise, have high charge mobility and good processability. The materials should be easily processable to form thin and large-area films for use in semiconductor devices. Other aims of the invention are immediately evident to those skilled in the art from the following description.

It has been reported in the literature that benzo[1,2-b:4,5-b'] dithiophene, hereinafter also shortly referred to as benzodithiophene with the following structure 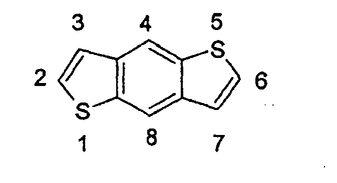 has a high charge carrier mobility and is useful as organic semiconductor. Benzodithiophene monomers or dimers, oligo- or polymers formed thereof and their use as an organic semiconductors have been described for example in Kossmehl et al., *Makromol. Chem.* 1983, **184(3)**, 627-50, Katz et al., *J. Mater. Chem.* 1997, **7(3),** 369-76, Laquindanum et al., *Adv. Mater.* 1997, **9(1),** 36-39 and in US 5,625,199.

**WO 98/04544 A1** discloses 4,5(or 6),7-trifluoro-benzo-b-thiophen-2-yl-carbonyl compounds of formula (1), wherin the groups Z independently represent hydrogen, cyano or a polymerisable group, suitable for use as such or mixed with other liquid crystal compounds in optical display elements or data storage means, in polarisers or in liquid crystal dyes.

Kobayashi K et al., "Crystal structures and physico-chemical properties of thiophene-fused TCNQ acceptors and their TTF complexes", International Conference on Science and Technology of Synthetic Metals (ICSM '88), Santa Fe, NM, USA, 26 June-2 July 1988, Vol. 27, no. 3-4, pages B309 - B314, ISSN 0379-6779, discloses isomeric benzodithiophene analogues of tetracyanoquindimethane.

In particular the dimeric bisbenzodithiophene has been shown to exhibit high charge carrier mobilities of 0.04 cm²V⁻¹s⁻¹. Its structure has flatter conformation than e.g. α-sexithiophene with comparable size. This enables compressed molecular packing and strong intermolecular interactions, which is favourable for compact stacking of the material and results in π-π-overlap and hence makes this compound an effective charge transport materials with high carrier mobilities. However, bisbenzodithiophene has a very high melting point over 400 °C and very low solubility in organic solvents, so that it cannot be readily solution processed and can only be vacuum deposited.

Therefore, another aim of the invention was to provide benzodithiophenes that are more easily processible in the manufacture of semiconductor devices.

It was found that the above aims can be achieved by providing reactive mesogenic benzodithiophenes, hereinafter also referred to as reactive benzodithiophene mesogens, according to the present invention. They consist of a central mesogenic core comprising one or more benzodithiophene groups, and optionally comprising further unsaturated organic groups that form a conjugated system together with the benzodithiophene groups, said mesogenic core being linked, optionally via a spacer group, to one or more reactive groups. The reactive mesogenic benzodithiophenes can induce or enhance liquid crystal phases or are liquid crystalline themselves. They can be oriented in their mesophase and the polymerisable group can be polymerised or crosslinked in situ to form polymer films with a high degree of order, thus yielding improved semiconductor materials with high stability and high charge carrier mobility.

A further aspect of the invention relates to liquid crystal polymers, in particular liquid crystal side chain polymers obtained from the reactive mesogenic benzodithiophenes according to the present invention, which are then further processed e.g. from solution as thin layers for use in semiconductor devices.

### Definition of Terms

The term 'liquid crystal or mesogenic material' or 'liquid crystal or mesogenic compound' should denote materials or compounds comprising one or more rod-shaped, board-shaped or disk-shaped mesogenic groups, i.e. groups with the ability to induce liquid crystal phase behaviour. Liquid crystal compounds with rod-shaped or board-shaped groups are also known in the art as 'calamitic' liquid crystals. Liquid crystal compounds with a disk-shaped group are also known in the art as 'discotic' liquid crystals. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerised.

The term 'reactive group' or 'reactive compound' includes compounds or groups that are capable of participating in a polymerisation reaction, like radicalic or ionic polymerisation from unsaturated functionality, polyaddition or polycondensation, as well as compounds or groups that are capable of being grafted for example by condensation or addition to a reactive polymer backbone in a polymeranaloguous reaction.

The term 'film' includes self-supporting, i.e. free-standing, films that show more or less pronounced mechanical stability and flexibility, as well as coatings or layers on a supporting substrate or between two substrates.

### Summary of the Invention

The invention relates to reactive mesogenic benzodithiophenes, consisting of a central mesogenic core that comprises one or more benzodithiophene groups and optionally comprises further unsaturated organic groups that form a conjugated system together with the benzodithiophene groups, said mesogenic core being linked, optionally via a spacer group, to one or more reactive groups.

The invention also relates to the use of reactive mesogenic benzodithiophenes as semiconductors or charge transport materials, in particular in optical, electro-optical or electronic devices, like for example in field effect transistors as components of integrated circuitry, as thin film transistors in flat panel display applications or RFID tags, or in semi-conducting components for organic light emitting diode (OLED) applications such as electroluminescent displays or backlights of flat panel displays, for photovoltaic or sensor devices, or as light-modulating components for liquid crystal displays, optical films or other optical or electrooptical devices.

The invention also relates to a field effect transistor, for example as a component of integrated circuitry, as a thin film transistor in flat panel display applications, or in an RFID tag, comprising one or more reactive or polymerised mesogenic benzodithiophenes according to the present invention.

The invention also relates to a semi-conducting component, for example in OLED applications like electroluminescent displays or backlights of flat panel displays, in photovoltaic or sensor devices, comprising one or more reactive or polymerised mesogenic benzodithiophenes according to the present invention.

### Detailed Description of the Invention

The reactive benzodithiophenes according to the present invention provide several advantages over prior art materials
- by adding substituent chains and other groups to the benzodithiophene core they can be made more soluble, thus being suitable for spin coating or solution coating techniques, rather than vacuum deposition, to prepare thin films for use e.g. in electronic devices such as transistors,
- they can be made mesogenic or liquid crystalline, thus exhibiting a higher degree of order that leads to particularly high charge carrier mobility, in particular when being aligned in their mesophase into macroscopically ordered orientation
- their macroscopic mesophase properties can be frozen in by in situ polymerisation,
- they combine the properties of a semi-conducting material with those of a mesogenic material to give novel materials with a rigid, planar conjugated core and a flexible chain to increase solubility and to decrease the melting point, which show high charge carrier mobility when being aligned in their mesophase.

The inventive reactive mesogenic benzodithiophenes are useful as charge transport semiconductors, in that they have high carrier mobilities. In particular, the introduction of side groups to the conjugated rings bonded to the benzodithiophene core improves their solubility and therefore their solution processability. In the compounds according to the present invention, the benzodithiophene group is a mesogenic group or part of a mesogenic group. These compounds are therefore particularly useful as semiconductors or charge transport materials, as they can be processed while in the highly ordered mesophase morphology, and readily aligned by conventional techniques in a preferred direction. Both smectic and nematic mesophase ordering allows close packing of molecular pi-electron systems, which maximises intermolecular charge transfer which occurs through a hopping mechanism between adjacent molecules. This ordered, and oriented microstructure can be permanently "frozen-in" by polymerising the mesogens, which can also create a structure with long range order, or "monodomain". Formation of a monodomain also maximises charge transfer by eliminating charge trap sites at grain boundaries, while the polymerisation also improves the mechanical properties of the film. Further, by cross-linking the mesogens, a highly stable structure results, which has an additional advantage of being impervious to subsequent processing solvents during device fabrication, thus allowing a wider range of solvents to be used in deposition of the next layer of the device by solution techniques. In addition, it is often observed that this cross-linking further densifies the film, leading to smaller intermolecular distances and improved charge transport.

It is also possible to copolymerise benzodithiophenes of the present invention with other mesogenic or liquid crystal monomers that are known from prior art, or with other reactive benzodithiophenes of the present invention, in order to induce or enhance liquid crystal phase behaviour.

Thus, another aspect of the invention relates to a reactive liquid crystal mixture comprising one or more reactive benzodithiophenes of the present invention, and optionally comprising one or more further reactive compounds, wherein at least one of the reactive benzodithiophenes and the further reactive compounds is mesogenic or liquid crystalline.

Particularly preferred are reactive mesogenic benzodithiophenes of the present invention, or mixtures comprising one or more reactive benzodithiophenes of the present invention, that exhibit a liquid crystal phase, especially a nematic and/or smectic liquid crystal phase.

Another aspect of invention relates to an anisotropic polymer film with charge transport properties obtainable from a reactive liquid crystal mixture as defined above that is aligned in its liquid crystal phase into macroscopically ordered orientation and polymerised or cross-linked to fix the oriented state.

Another aspect of the invention relates to a liquid crystal side chain polymer (SCLCP) obtained from a reactive liquid crystal material as defined above by polymerisation or polymeranaloguous reaction. Particularly preferred are SCLCPs obtained from one or more reactive benzodithiophenes or from a reactive mixture comprising one or more benzodithiophenes as described above.

Another aspect of the invention relates to an SCLCP obtained from one or more reactive benzodithiophenes or from a reactive liquid crystal mixture as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 Å) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

The invention also relates to the use of reactive mesogenic benzodithiophenes of the present invention, or liquid crystal mixtures or polymers obtained thereof, as light-modulating component in liquid crystal displays, which may for example be switchable between two different states by an electric field, for components of liquid crystal displays, in particular optical retardation or compensation films, alignment layers or polarisers, or in other optical or electrooptical devices.

The invention also relates to a liquid crystal display, component of a liquid crystal display, in particular an optical retardation or compensation films, alignment layer or polariser, or an other optical or electrooptical device comprising reactive benzodithiophenes according to the present invention, or liquid crystal mixtures or polymer films obtained thereof.

The benzodithiophene groups in the compounds of the present invention are preferably linked to their neighbouring groups at the 2- and 6-position.

Especially preferred are compounds selected of formula I

P-Sp-X-T-R I

wherein
- P: is a polymerisable or reactive group,
- Sp: is a spacer group or a single bond,
- X: is a linkage group or a single bond,
- R: is H, halogen, CN, NO₂, an aliphatic, alicyclic or aromatic group with up to 40 C atoms that optionally comprises one or more hetero atoms and wherein one or more rings can be fused, or P-Sp-X-, and
- T: is a mesogenic group comprising one or more benzodithiophene groups that are substituted or unsubstituted.

R in formula I is preferably H, F, Cl or straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CY¹=CY²- or -C≡C- in such a manner that O and/or S atoms are not linked directly to one another, or an aromatic or heteroaromatic group, with Y¹ and Y² being independently of one another H, F, Cl or CN.

Particularly preferably R is optionally fluorinated alkyl or alkoxy with 1 to 15 C atoms.

Further preferred are compounds of formula I wherein R is P-Sp-X-.

T in formula I preferably comprises 1 or 2 benzodithiophene groups.

Particularly preferably T is selected of formula II

-Z¹-(A¹-Z²)ₘ-(T¹-Z³)ₙ-(A²-Z⁴)ₒ- II

wherein
- A¹ and A²: are independently of each other an aromatic, heteroaromatic, or alicyclic group with up to 18 C atoms which is unsubstituted, mono- or polysubstituted with R¹, and A¹ may also denote T¹,
- Z¹ to Z⁴: are independently of each other -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C-, -CH=CH-COO-, -OCO-CH=CH- or a single bond,
- Y¹ and Y²: are independently of each other H, F, Cl or CN,
- T¹: is a group consisting of 1 or 2 benzodithiophene units which are optionally substituted by R¹,
- R¹: is H, halogen, CN, NO₂, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CY¹=CY²- or -C≡C- in such a manner that O and/or S atoms are not linked directly to one another, an aromatic or heteroaromatic group, or P-Sp-X,
- R⁰ and R⁰⁰: are independently of each other H or alkyl with 1 to 12 C-atoms,
- m and o: are independently of each other 0, 1, 2 or 3, and
- n: is 1, 2 or 3

Particularly preferred groups T are those wherein Z¹, A¹, Z², T¹, Z³, A² and Z⁴ form a conjugated system. Therein A¹ and A² are preferably arylene or heteroarylene and Z¹, Z², Z³ and Z⁴ are preferably a single bond or a conjugated link such as -CY¹=CY²- or -C≡C-.

Further preferred groups T are those wherein m and o are 0, further those wherein m and o are 1 or 2.

Further preferred groups T are those wherein T¹ is benzodithiophene that is optionally substituted with R¹ as defined above, furthermore those wherein n is 1 or 2 and Z² is a single bond or a conjugated link such as -CY¹=CY²- or -C≡C-.

Particularly preferred groups T are those of the following formulae

-Z¹-T¹-Z³- II1

-Z¹-A¹-Z²-T¹-Z³- II2

-Z¹-T¹-Z³-T¹-Z³- II3

-Z¹-A¹-Z²-T¹-Z³-A²-Z⁴- II4

-Z¹-A¹-Z²-A¹-Z²-T¹-Z³- II5

-Z¹-A¹-Z²-T¹-Z³-T¹-Z³- II6

-Z¹-T¹-Z²-A¹-Z²-T¹-Z³- II7

-Z¹-A¹-Z²-A¹-Z²-T¹-Z³-A²-Z⁴- II8

-Z¹-A¹-Z²-A¹-Z²-A¹-Z²-T²-Z³- II9

-Z¹-A¹-Z²-A¹-Z²-T¹-Z³-T²-Z³- II10

-Z¹-A¹-Z²-T¹-Z²-A¹-Z²-T²-Z³- II11

-Z¹-A¹-Z²-T¹-Z³-T¹-Z³-A²-Z⁴- II12

-Z¹-T¹-Z²-A¹-Z²-A¹-Z²-T²-Z³- II13

-Z¹-A¹-Z²-T¹-Z³-T¹-Z³-T¹-Z³- II14

-Z¹-T¹-Z²-A¹-Z²-T¹-Z³-T¹-Z³- II15

-Z¹-A¹-Z²-A¹-Z²-A¹-Z²-A¹-Z²-T¹-Z³- II16

-Z¹-A¹-Z²-A¹-Z²-A¹-Z²-T¹-Z³-A¹-Z⁴- II17

-Z¹-A¹-Z²-A¹-Z²-T¹-Z³-A²-Z⁴-A²-Z⁴ II18

-Z¹-A¹-Z²-A¹-Z²-A¹-Z²-T¹-Z³-T¹-Z³- II19

-Z¹-A¹-Z²-A¹-Z²-T¹-Z²-A¹-Z²-T¹-Z³- II20

-Z¹-A¹-Z²-T¹-Z²-A¹-Z²-A¹-Z²-T¹-Z³- II21

-Z¹-A¹-Z²-A¹-Z²-T¹-Z³-T¹-Z³-A²-Z⁴- II22

-Z¹-A¹-Z²-T¹-Z²-A¹-Z²-T¹-Z³-A²-Z⁴- II23

-Z¹-T¹-Z²-A¹-Z²-A¹-Z²-A¹-Z²-T¹-Z³- II24

-Z¹-A¹-Z²-A¹-Z²-T¹-Z³-T¹-Z³-T¹-Z³- II25

-Z¹-A¹-Z²-T¹-Z²-A¹-Z²-T¹-Z³-T¹-Z³- II26

-Z¹-A¹-Z²-T¹-Z²-T¹-Z²-A¹-Z²-T¹-Z³- II27

-Z¹-A¹-Z²-T¹-Z³-T¹-Z³-T¹-Z³-A²-Z⁴- II28

-Z¹-T¹-Z²-A¹-Z²-T¹-Z²-A¹-Z²-T¹-Z³- II29

-Z¹-T¹-Z²-A¹-Z²-A¹-Z²-T¹-Z³-T¹-Z³- II30

wherein Z¹, Z², Z³, Z⁴, A¹, A² and T¹ have in each case independently one of the meanings of formula II.

T¹ is preferably benzo[1,2-b:4,5-b']thiophene-2,6-diyl or [2,2']-bibenzo[1,2-b:4,5-b']thiophene-6,6'-diyl, all of which are optionally mono- or polysubstituted by halogen, CN, NO₂, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CY¹=CY²- or -C≡C- in such a manner that O and/or S atoms are not linked directly to one another, an aromatic or heteroaromatic group, or P-Sp-X.

T¹ is preferably selected from the following subformulae 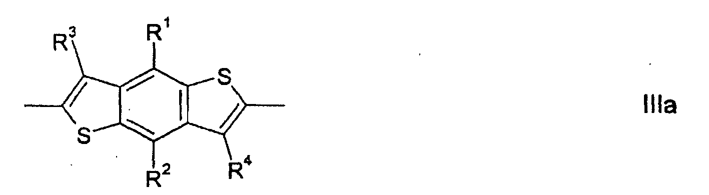 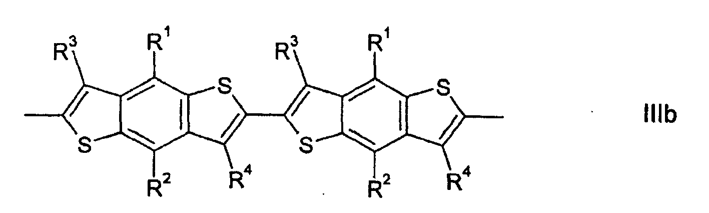 wherein R¹ to R⁴ have independently of one another one of the meanings of R¹ in formula II, and are preferably H, halogen, methyl, ethyl, propyl, CO₂Me, CO₂Et, CN, COCH₃ or CHO.

A¹ and A² are preferably selected from 1,4-phenylene, 1,4-cyclohexa-1,3-diene, 1,4-cyclohexenylene in which, in addition, one or more CH groups are optioanlly replaced by N or one or two non-adjacent CH₂ groups are optionally replaced by O and/or S, thiophene-2,5-diyl, thienothiophene-2,5-diyl, dithienothiophene-2,6-diyl, 1,4-bicyclo-(2,2,2)-octylene, naphthalene-2,6-diyl, furan 2,5 diyl, and indane-2,5-diyl, wherein all these groups are unsubstituted, mono- or polysubstituted by L, with L being F, Cl, Br, CN, SCN, NO₂, SF₅ or an alkyl, alkoxy, alkylcarbonyl or alkoxycarbonyl group with 1 to 12 C atoms, wherein one or more H atoms are optionally replaced by F or Cl.

A¹ and A² are particularly preferably 1,4-phenylene that is substituted with 1, 2 or 3 groups L as defined above, or thiophene-2,5-diyl, all of which are optionally substituted with one or more groups L as defined above.

Z¹⁻⁴ are preferably selected from -O-. -S-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C=C- and a single bond, in particular from -CH=N-, -N=CH-, -N=N-, -CH=CR⁰-, -CY¹=CY²-, -C≡C- and a single bond.

Particularly preferred are the following compounds 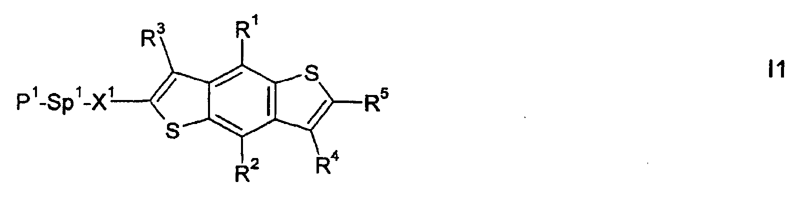 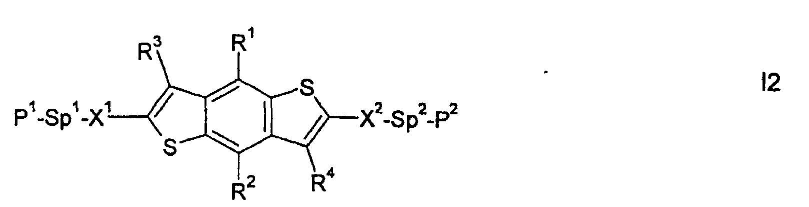 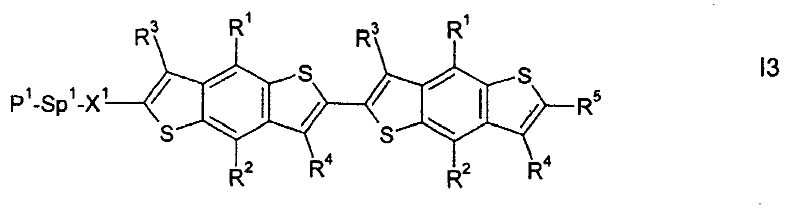 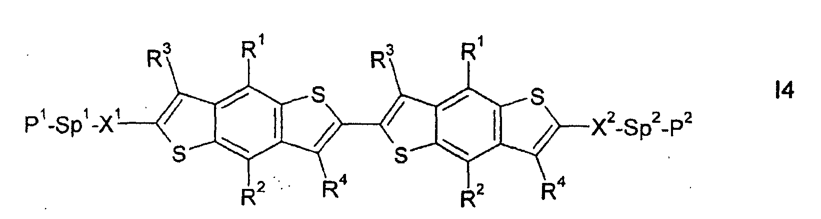 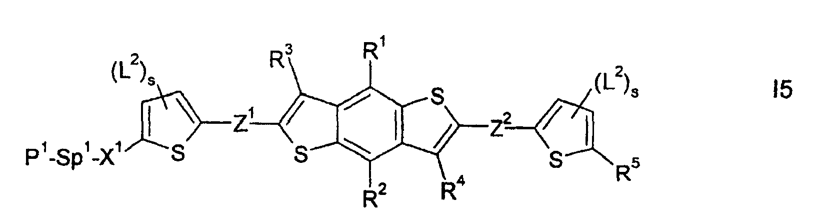 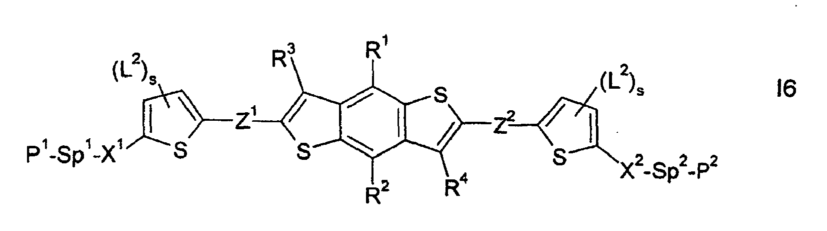 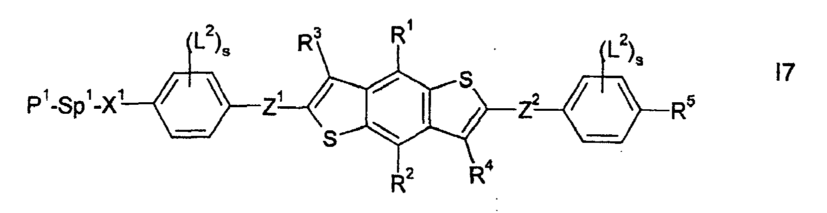 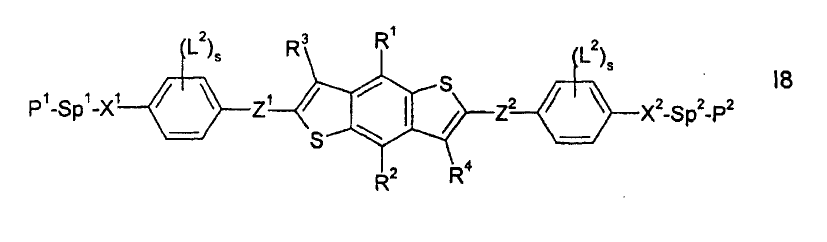 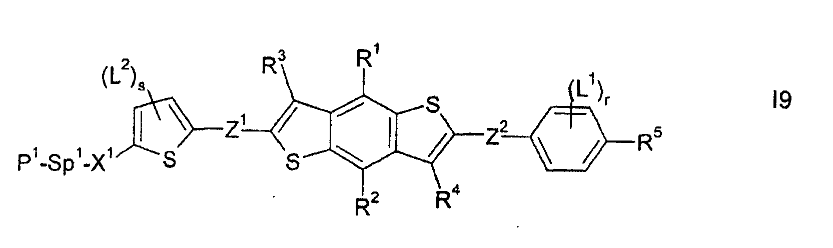 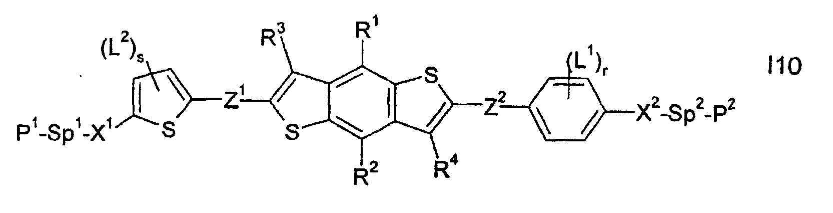 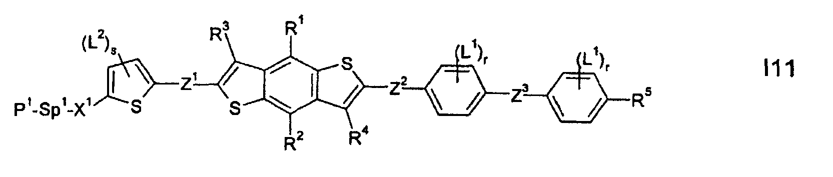 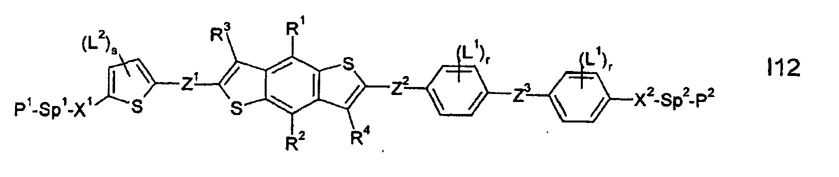 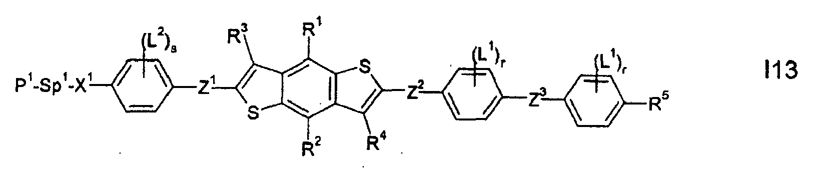 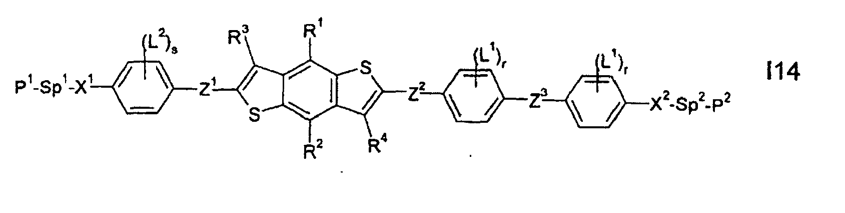 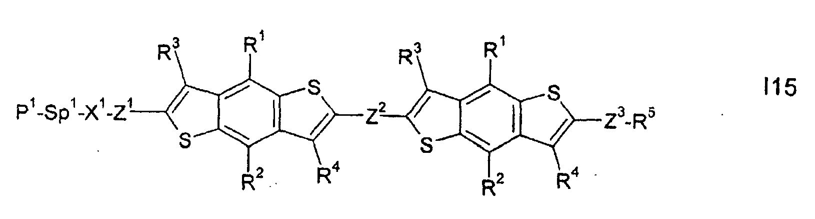 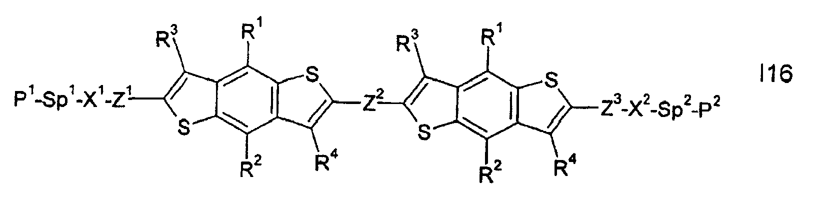 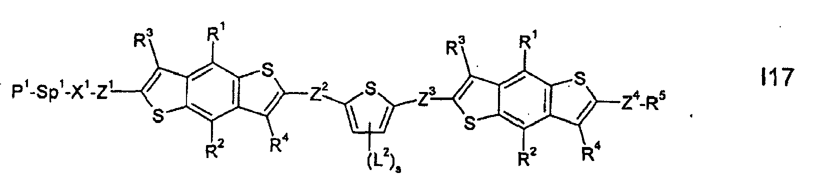 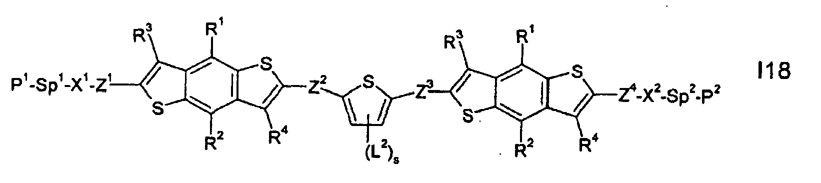 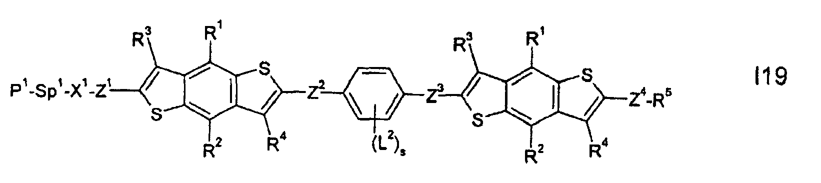 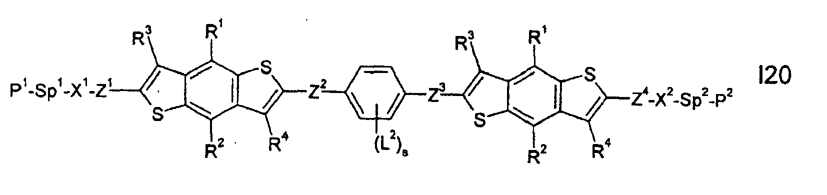 wherein
- P¹ and P²: are identical or different groups P as defined in formula I,
- Sp¹ and Sp²: are identical or different groups Sp as defined in formula I,
- X¹ and X²: are identical or different groups X as defined in formula I,
- Z¹ to Z⁴: are as defined in formula II, and are preferably -CH=CH-, -CH=CF-, -CF=CH-, CH=CCl-, -CCl=CH-, -CF=CF-, -CCl=CCl-, -C≡C- or a single bond,
- R¹ to R⁴: have independently of one another the meaning of R¹ in formula II, and are preferably halogen or an optionally fluorinated alkyl group with 1 to 15 C atoms,
- R⁵: has one of the meanings of R¹ given in formula II, and is preferably halogen or an optionally fluorinated alkyl group with 1 to 15 C atoms,
- L¹: has one of the meanings of L given above, and is preferably F, Cl or alkyl or alkoxy with 1 to 3 C-atoms that is optionally mono-, poly- or perfluorinated,
- L²: has one of the meanings of L given above, and is preferably alkyl with 1 to 12 C-atoms that is optionally mono-, poly- or perfluorinated,
- r: is 0, 1, 2, 3 or 4, and
- s: is 0, 1 or 2.

If in the above formulae a group X^{1,2} is adjacent to a group Z¹⁻⁴, preferably at least one of X^{1,2} and Z¹⁻⁴ is a single bond.

In the foregoing and the following, arylene and heteroarylene preferably denote a bivalent mono-, bi- or tricyclic aromatic or heteroaromatic group with up to 25 C atoms that optionally comprises fused rings and wherein the heteroaromatic groups contain at least one hetero atom, preferably selected from N, O and S, and which in each case is optionally substituted with one or more groups selected from H, halogen and straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another.

Very preferred arylene and heteroarylene groups are those having one of the preferred meanings of A¹ as given above and below.

Aryl and heteroaryl preferably denote a mono-, bi- or tricyclic aromatic or heteroaromatic group with up to 25 C atoms that optionally comprises fused rings and wherein the heteroaromatic groups contain at least one hetero atom, preferably selected from N, O and S, and which in each case is optionally substituted with one or more groups selected from H, halogen and straight chain, branched or cyclic alkyl with 1 to 20 C-atoms, which is unsubstituted, mono- or poly-substituted by F, Cl, Br, I or CN, and in which one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another.

Especially preferred aryl and heteroaryl groups are phenyl in which, in addition, one or more CH groups are optionally replaced by N, naphthalene, thiophene, thienothiophene, dithienothiophene, alkyl fluorene and oxazole, all of which are unsubstituted, mono- or polysubstituted with L, wherein L is halogen or an alkyl, alkoxy, alkylcarbonyl or alkoxycarbonyl group with 1 to 12 C atoms, wherein one or more H atoms are optionally replaced by F or Cl.

Further preferred aryl and heteroaryl groups include five-membered heterocyclics like oxazole or isoxazole, N-substituted imidazole or pyrazole, thiazole or isothiazole, oxadiazole, N-substituted triazole, six-membered heterocyclics like pyridine, pyridazine, pyrimidine, pyrazine, triazine and tetrazine, heterocyclics with fused rings like benzoxazole, benzothiazole, benzimidazole, quinoline, isoquinoline, cinnoline, quinazoline, quinoxaline, phthalazine, benzothiadiazole, benzotriazole, benzotriazine, phenazine, phenanthridine, acridine, or condensed polycyclics like acenaphthene, phenanthrene, anthracene, fluoranthene, pyrene, perylene, rubrene, chrysene, naphthacene, coronene or triphenylene, all of which are unsubstituted, mono- or polysubstituted with L as defined above.

-CY¹=CY²- is preferably -CH=CH-, -CH=CF-, -CF=CH-, -CF=CF-, -CH=C(CN)- or -C(CN)=CH-.

If one of R¹ to R⁵ is an alkyl or alkoxy radical, i.e. where the terminal CH₂ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Halogen is preferably F or Cl.

Hetero atoms are preferably selected from N, O and S.

The polymerisable or reactive group P is preferably selected from CH₂=CW¹-COO-, 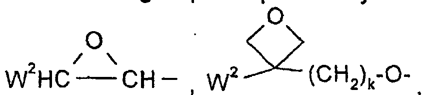 CH₂=CW²⁻(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferred groups P are CH₂=CH-COO-, CH₂=C(CH₃)-COO-, CH₂=CH-, CH₂=CH-O-, (CH₂=CH)₂CH-OCO-, (CH₂=CH)₂CH-O-, and

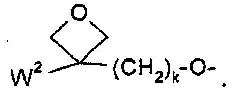

Very preferred are acrylate and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp all groups can be used that are known for this purpose to those skilled in the art, and is preferably linear or branched alkylene group having 1 to 20 C atoms, in particular 1 to 12 C atoms, in which, in addition, one or more non-adjacent CH₂ groups may be replaced by -O-, -S-, -NH-, -N(CH₃)-, -CO-, -O-CO-, -S-CO-, -O-COO-, -CO-S-, -CO-O-, -CH(halogen)-, -C(halogen)₂, -CH(CN)-, -CH=CH- or -C≡C-, or a siloxane group.

Typical spacer groups are for example -(CH₂)ₚ-, -(CH₂CH₂O)ᵣ-CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, r being an integer from 1 to 3 and R⁰ and R⁰⁰ having the meanings given in formula I.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyliminoethylene, 1-methylalkylene, ethenylene, propenylene and butenylene for example.

The linkage group X is preferably -O-, -S-, -OCH₂-, -CH₂O-, -CO-, -COO-, -OCO-, -OCO-O-, -CO-NR⁰-, -NR⁰-CO-, -OCH₂-, -CH₂O-, -SCH₂-, -CH₂S-, -CH=CH-COO-, -OOC-CH=CH-, -CF₂O-, -OCF₂-, -CF₂S-, -SCF₂- -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C- or a single bond, with R⁰, Y¹ and Y² having the meanings given above.

In a preferred embodiment, the linkage group X is an unsaturated group that is capable of forming a conjugated system, such as -CH=N-, -N=CH-, -N=N-, -CH=CR⁰-, -CY¹=CY²-, -C≡C-, or a single bond.

Further preferred are compounds with one or two groups P-Sp-X wherein Sp and/or X is a single bond.

In case of compounds with two or more groups P-Sp-X, each of the groups P, the groups Sp, and the groups X can be identical or different.

SCLCPs obtained from the inventive compounds or mixtures by polymerisation or copolymerisation have a backbone that is formed by the polymerisable group P in formula I.

The compounds of formula I can be synthesized according to or in analogy to methods that are known to the skilled in the art and are reported in the literature. Furthermore, they can be prepared according to or in analogy to the following reaction schemes.

As shown in Scheme 1, fused benzodithiophene ring system 1 and its dimer 2 can be prepared as described e.g. in Katz et al., *J. Mater. Chem.* 1997, **7(3),** 369-76, or Yoshida et al., *J. Org. Chem.* 1994, **59,** 3077.

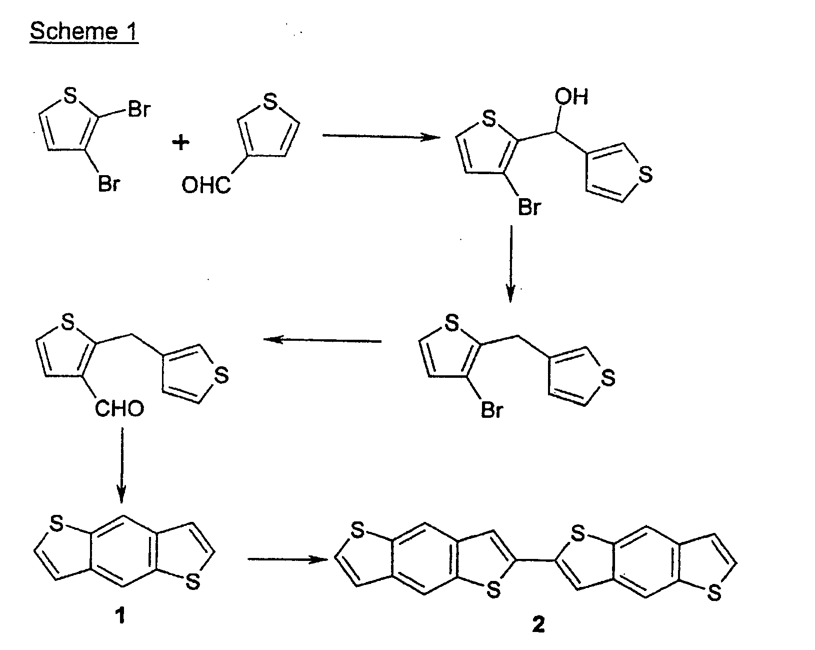

As shown in scheme 2, compound 1 can be lithiated and reacted with express iodine to give di-iododerivative 3, as described e.g. in Yoshida et al., *J. Org. Chem.* 1994, **59,** 3077, and further reacted with acetylene compounds using the Sonogashira reaction to give compounds of type 5.

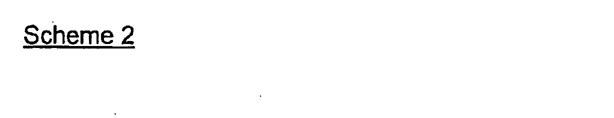

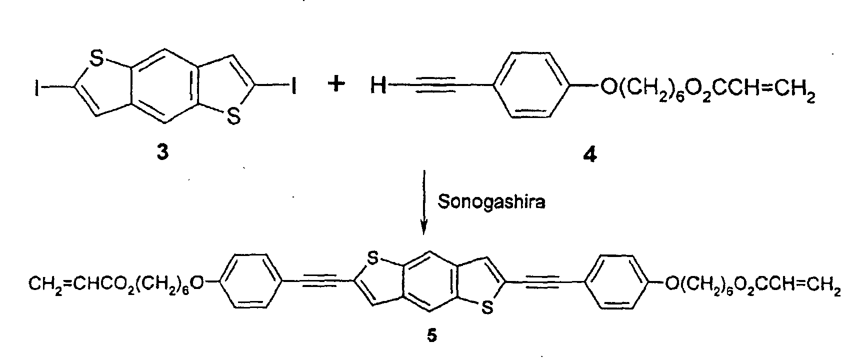

As shown in Scheme 3, compound 3 can be cross-coupled with boronic acids according to the Suzuki reaction to give compounds of type 7.

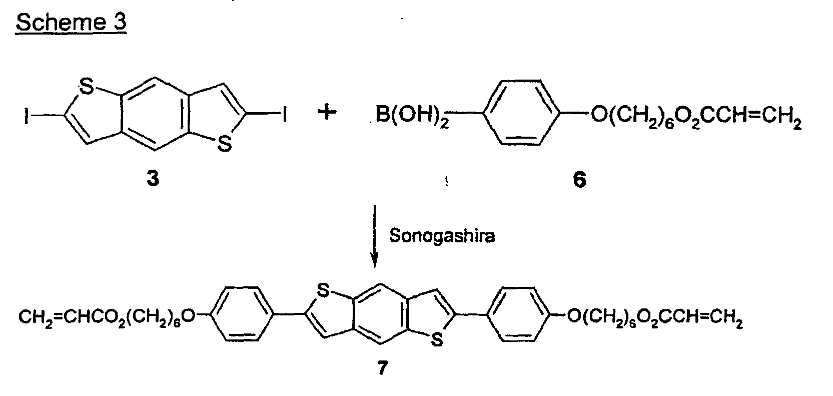

As shown in Scheme 4, reaction with one equivalent of boronic acid and Suzuki conditions followed by isolation gives compound 8. Reaction of an acetylene moiety gives an unsymmetrical compound of type 9.

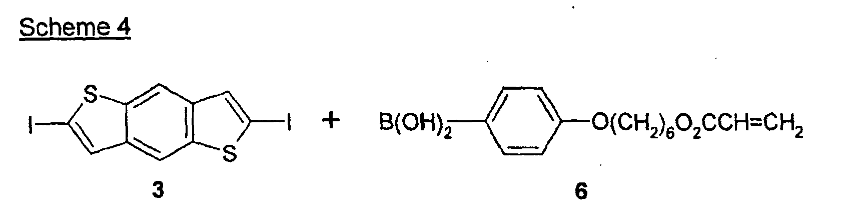

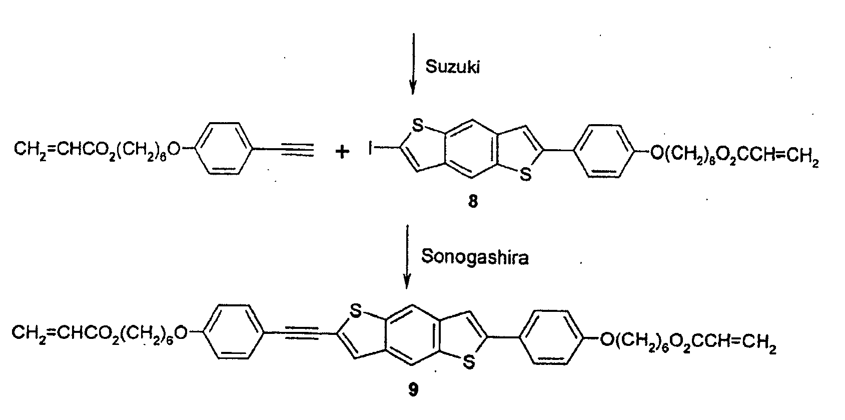

As shown in Scheme 5, compound 10 formed from a Sonogashira cross-coupling can be reacted with a boronic acid ester to give compounds of type 12.

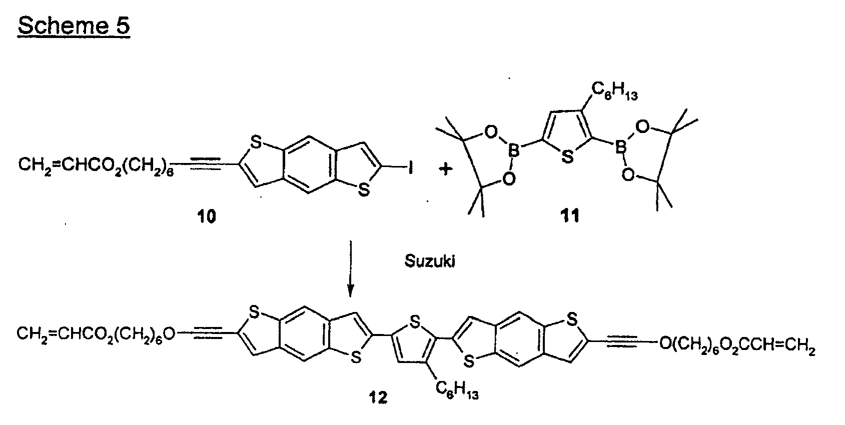

In the compounds shown in Scheme 1-5 above, the central benzene ring of the benzodithiophene group can be easily substituted with alkyl or alkoxy groups, as described e.g. in Beimling et al., *Chem. Ber.* 1986, **119(10)**, 3198.

A preferred embodiment of the present invention relates to reactive benzodithiophenes, in particular those of formula I, that are mesogenic or liquid crystalline. These materials are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

It is also possible to copolymerise the benzodithiophenes according to the present invention with other polymerisable mesogenic or liquid crystal monomers that are known from prior art, in order to induce or enhance liquid crystal phase behaviour.

Thus, another aspect of the invention relates to a polymerisable liquid crystal material comprising one or more polymerisable benzodithiophenes of the present invention as described above and below comprising at least one polymerisable group, and optionally comprising one or more further polymerisable compounds, wherein at least one of the polymerisable benzodithiophenes of the present invention and/or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred.

Another aspect of the invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Polymerisation is preferably carried out by in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Polymerisable benzodithiophenes comprising one or more groups P-Sp-X can also be copolymerised with polymerisable mesogenic compounds to induce, or, in case of mesogenic materials of formula I, enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

SCLCPs can be prepared from the polymerisable compounds or mixtures according to the invention by the methods described above, or by conventional polymerisation techniques which are known to those skilled in the art, including for example radicalic, cationic or anionic polymerisation from unsaturated functionality radicalic, polyaddition or polycondensation. Polymerisation can be carried out for example as polymerisation in solution, without the need of coating and prior alignment, or polymerisation in situ.

It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added e.g. to polysiloxane backbones with Si-H groups.

It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397; EP 0,261,712; DE 195,04,224; WO 95/22586 and WO 97/00600. Typical non mesogenic comonomers are for example alkyl mono- or diacrylates or alkyl mono- or dimethacrylates with alkyl groups of 1 to 20 C atoms, like methyl acrylate or methyl methacrylate, trimethylpropane trimethacrylate or pentaerythritol tetraacrylate.

For example, if a device is made from a polymerisable liquid crystal material by polymerisation in situ, the liquid crystal material preferably comprises one or more compounds of formula I and its preferred subformulae having one or more groups P. If a liquid crystal polymer is prepared first, for example by polymerisation in solution, and the isolated polymer is used to make the device, the polymer is preferably made from a liquid crystal material comprising one or more compounds of formula I and its preferred subformulae having one group P.

The materials of the present invention are useful as optical, electronic and semiconductor materials, in particular as charge transport materials in field effect transistors (FETs) e.g. as components of integrated circuitry, ID tags or TFT applications. Alternatively, they may be used in organic light emitting diodes (OLEDs) in electroluminescent display applications or as backlight of e.g. liquid crystal displays, as photovoltaics or sensor materials, for electrophotographic recording, and for other semiconductor applications.

Especially the oligomers and polymers according to the invention show advantageous solubility properties which allow production processes using solutions of these compounds. Thus films, including layers and coatings, may be generated by low cost production techniques e.g. spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known e.g. from US 5,892,244, WO 00/79617, US 5,998,804, and from the references cited in the background and prior art chapter and listed below. Due to the advantages, like low cost production using the solubility properties of the compounds according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the materials according to the invention may be used in organic light emitting devices or diodes (OLEDs), e.g. in display applications or as backlight of e.g. liquid crystal displays. Common OLEDs are realized using multilayer structures. An emission layer is generally sandwiched between one or more electron-transport and/or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/ or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g. of display devices such as described in EP 0 889 350 A1 or by C. Weder et al., Science, 279, 1998, 835-837.

The materials of the present invention are also useful for the preparation of optical films with anisotropic properties, like for example polarizers, optical retardation films, compensators, colour filters, polarization beam splitters, or polarization filters, which can be used for example as components of liquid crystal displays. Furthermore, they can be used as coatings e.g. for decorative or security use, as adhesives, or for the preparation of liquid crystal pigments.

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICl, ICl₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCl, HNO₃, H₂SO₄, HClO₄, FSO₃H and ClSO₃H), transition metal compounds (e.g. FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂lrCl₆, La(NO₃)₃ · 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns ot tracts in electronic applications such as printed circuit boards and condensers.

## Claims

1. Reactive mesogenic benzodithiophenes, consisting of a central mesogenic core that comprises one or more benzodithiophene groups and optionally comprises further unsaturated organic groups that form a conjugated system together with the benzodithiophene groups, said mesogenic core being linked, optionally via a spacer group, to one or more reactive groups.

2. Reactive mesogenic benzodithiophenes according to claim 1, **characterized in that** they are selected of formula I
P-Sp-X-T-R I
wherein
P is a polymerisable or reactive group,
Sp is a spacer group or a single bond,
X is a linkage group or a single bond,
R is H, halogen, CN, NO₂, an aliphatic, alicyclic or aromatic group with up to 40 C atoms that optionally comprises one or more hetero atoms and wherein one or more rings can be fused, or P-Sp-X-, and
T is a mesogenic group comprising one or more benzodithiophene groups that are substituted or unsubstituted.

3. Reactive mesogenic benzodithiophenes according to claim 2, **characterized in that** T is selected of formula II
-Z¹-(A¹-Z²)ₘ-(T¹-Z³)ₙ-(A²-Z⁴)ₒ- II
wherein
A¹ and A² are independently of each other an aromatic, heteroaromatic or alicyclic group with up to 18 C atoms which is unsubstituted, mono- or polysubstituted with R¹, and A¹ may also denote T¹,
Z¹ to Z⁴ are independently of each other -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C-, -CH=CH-COO-, -OCO-CH=CH- or a single bond,
Y¹ and Y² are independently of each other H, F, Cl or CN,
T¹ is a group consisting of 1 or 2 benzodithiophene units which are optionally substituted by R¹,
R¹ is H, halogen, CN, NO₂, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CY¹=CY²- or -C≡C- in such a manner that O and/or S atoms are not linked directly to one another, an aromatic or heteroaromatic group, or P-Sp-X,
R⁰ and R⁰⁰ are independently of each other H or alkyl with 1 to 12 C-atoms,
m and o are independently of each other 0, 1, 2 or 3, and
n is 1, 2 or 3

4. Reactive mesogenic benzodithiophenes according to claim 3, **characterized in that** T¹ is selected of the following subformulae 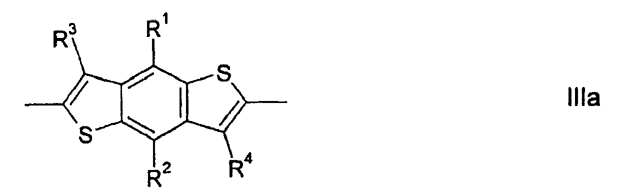 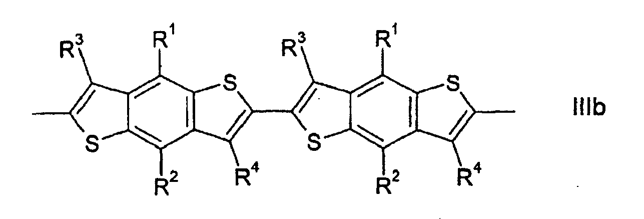 wherein R¹ to R⁴ are independently of one another H, halogen, CN, NO₂, straight chain, branched or cyclic alkyl with 1 to 20 C-atoms, which is unsubstituted, mono- or polysubstituted by F, Cl, Br, or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CY¹=CY²- or -C=C- in such a manner that O and/or S atoms are not linked directly to one another, an aromatic or heteroaromatic group, or P-Sp-X.

5. Reactive mesogenic benzodithiophenes according to claim 3 and/or 4, **characterized in that** A¹ and A² are selected from 1,4-phenylene, 1,4-cyclohexa-1,3-diene, 1,4-cyclohexenylene in which, in addition, one or more CH groups are optioanlly replaced by N or one or two non-adjacent CH₂ groups are optionally replaced by O and/or S, thiophene-2,5-diyl, thienothiophene-2,5-diyl, dithienothiophene-2,6-diyl, 1,4-bicyclo-(2,2,2)-octylene, naphthalene-2,6-diyl, furan 2,5 diyl, and indane-2,5-diyl, all these groups being unsubstituted, mono- or polysubstituted by L, with L being F, Cl, Br, CN, SCN, NO₂, SF₅ or an alkyl, alkoxy, alkylcarbonyl or alkoxycarbonyl group with 1 to 12 C atoms, wherein one or more H atoms are optionally replaced by F or Cl.

6. Reactive mesogenic benzodithiophenes according to at least one of claims 3 to 5, **characterized in that** they are selected from the following formulae 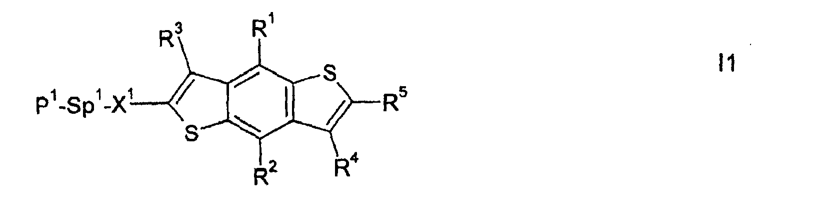 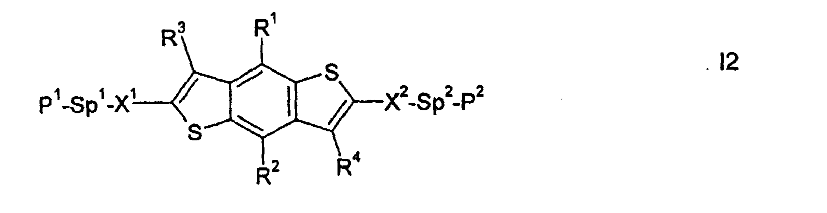 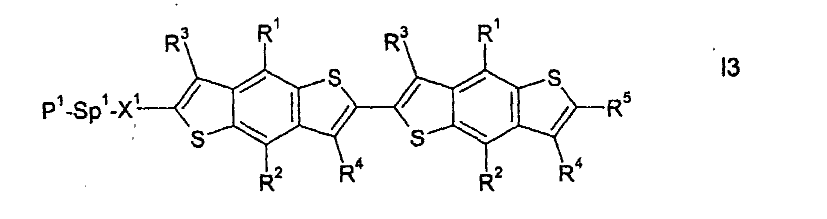 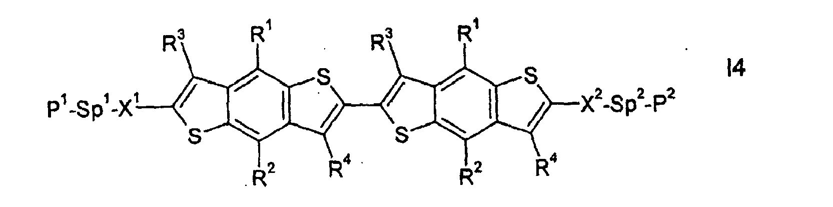 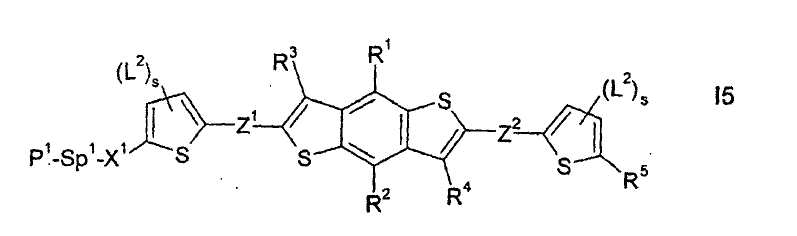 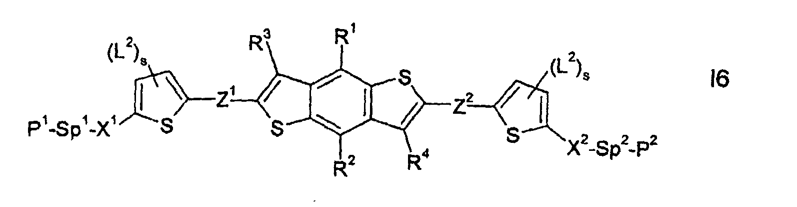 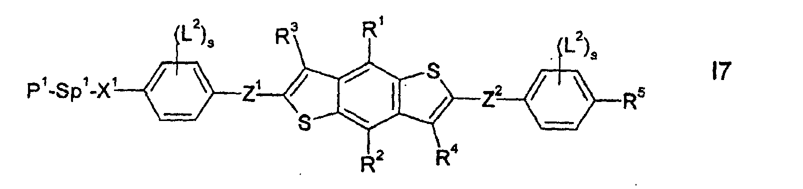 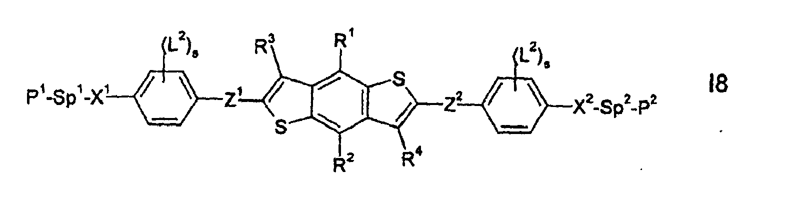 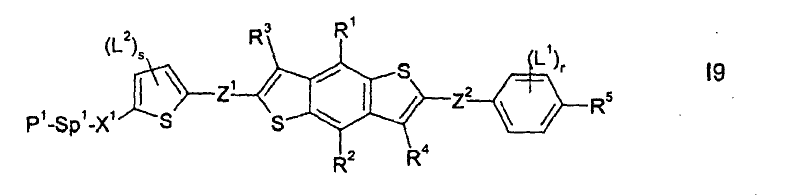 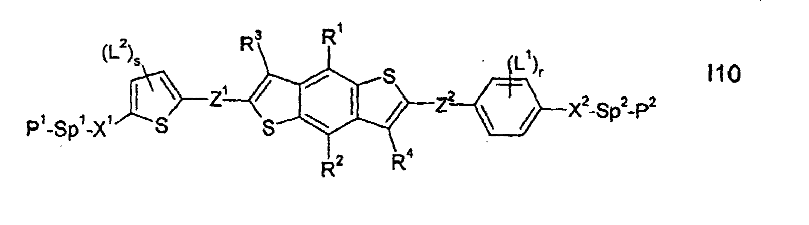 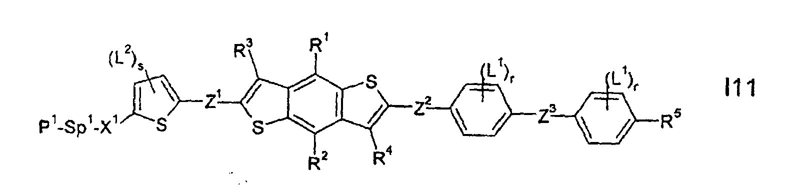 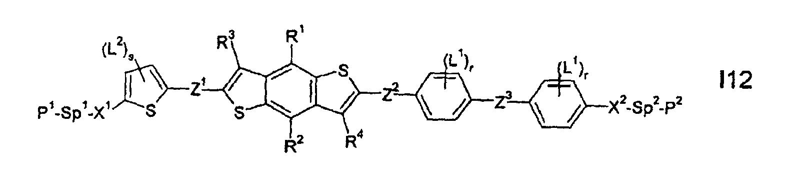 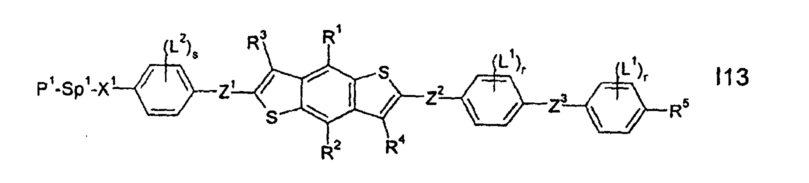 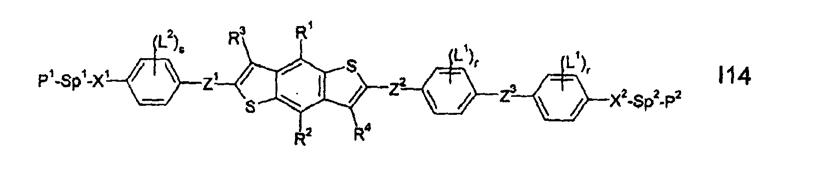 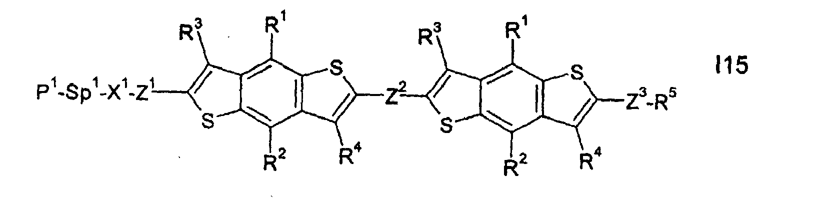 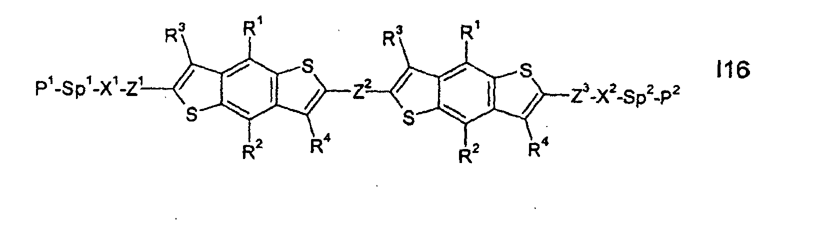 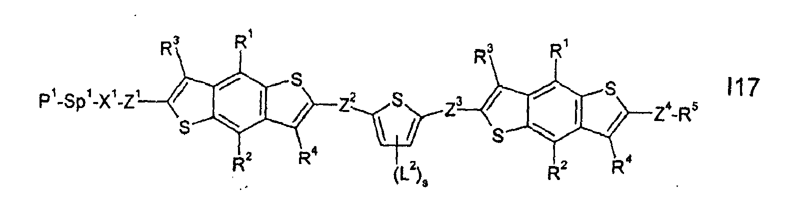 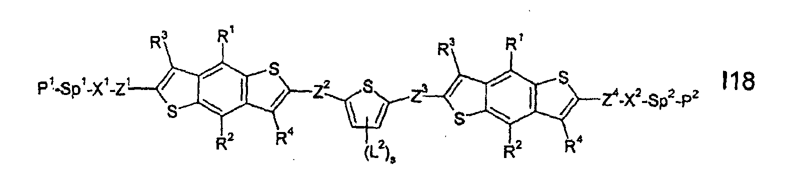 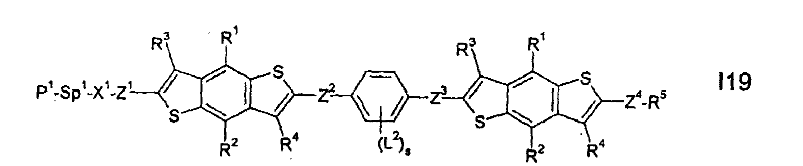 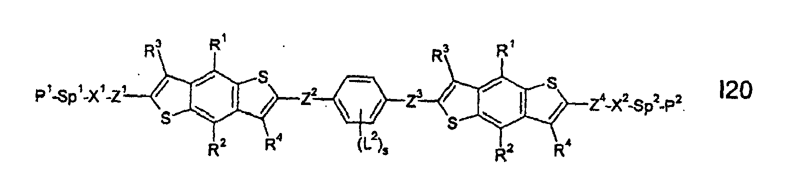 wherein
P¹ and P² are identical or different groups P as defined in claim 2,
Sp¹ and Sp² are identical or different groups Sp as defined in claim 2,
X¹ and X² are identical or different groups X as defined in claim 2,
Z¹ to Z⁴ are as defined in formula II, and are preferably -CH=CH-, -CH=CF-, -CF=CH-, CH=CCl-, -CCl=CH-, -CF=CF-, -CCl=CCl-, -C≡C- or a single bond,
R¹ to R⁴ have independently of one another one of the meanings of R¹ in formula II, and are preferably halogen or an optionally fluorinated alkyl group with 1 to 15 C atoms,
R⁵ has one of the meanings of R¹ in formula II, and is preferably halogen or an optionally fluorinated alkyl group with 1 to 15 C atoms,
L¹ has one of the meanings of L as defined in claim 5, and is preferably F, Cl or alkyl or alkoxy with 1 to 3 C-atoms that is optionally mono-, poly- or perfluorinated,
L² has one of the meanings of L as defined in claim 5, and is preferably alkyl with 1 to 12 C-atoms that is optionally mono-, poly- or perfluorinated,
r is 0, 1, 2, 3 or 4, and
s is 0, 1 or 2.

7. Reactive mesogenic benzodithiophenes according to at least one of claims 1 to 6, **characterized in that** the polymerisable or reactive group or group P is selected from CH₂=CW¹-COO-, 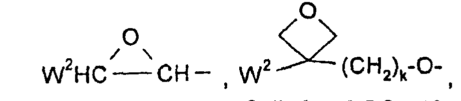 CH₂=CW²-(O)ₖ₁-, CH₃₋CH=CH-O-, (CH₂=CH)₂CH-OCO-, (CH₂=CH-CH₂)₂CH-OCO-, (CH₂=CH)₂CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

8. Reactive mesogenic benzodithiophenes according to at least one of claims 1 to 7, **characterized in that** they exhibit a liquid crystal phase.

9. Reactive liquid crystal mixture comprising one or more reactive mesogenic benzodithiophenes according to at least one of claims 1 to 8 and optionally one or more further reactive compounds, wherein at least one of said benzodithiophenes and further reactive compounds is mesogenic or liquid crystalline.

10. Anisotropic polymer film with charge transport properties obtainable from a reactive liquid crystal mixture according to claim 9 that is aligned in its liquid crystal phase into macroscopically uniform orientation and polymerised or crosslinked to fix the oriented state.

11. Side chain liquid crystal polymer obtained by polymerisation of one or more compounds or a reactive material according to claims 1 to 9 or by grafting one or more compounds or a reactive material according to claims 1 to 9 to a polymer backbone in a polymeranaloguous reaction, optionally with one or more additional mesogenic or non-mesogenic comonomers.

12. Use of the reactive mesogenic benzodithiophenes, polymerisable mixtures and polymers according to at least one of claims 1 to 11 as semiconductors or charge transport materials, in particular in optical, electrooptical or electronic devices, like for example components of integrated circuitry, field effect transistors (FET) for example as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors and for electrophotographic applications like electrophotographic recording, or as light-modulating components for liquid crystal displays, optical films or other optical or electrooptical devices.

13. Field effect transistor, for example as a component of integrated circuitry, as a thin film transistor in flat panel display applications, or in a Radio Frequency Identification (RFID) tag, comprising one or more reactive mesogenic benzodithiophenes, reactive mixtures or polymers according to at least one of claims 1 to 11.

14. Security marking or device comprising comprising one or more reactive mesogenic benzodithiophenes, reactive mixtures or polymers according to at least one of claims 1 to 11, or a FET or RFID tag according to claim 13.

15. Reactive mesogenic benzodithiophenes, reactive mixtures or polymers according to at least one of claims 1 to 11, which are oxidatively or reductively doped to form conducting ionic species.

16. Charge injection layer, planarising layer, antistatic film or conducting substrate or pattern for electronic applications or flat panel displays, comprising one or more reactive mesogenic benzodithiophenes, reactive mixtures or polymer films according to claim 15.

## Patentansprüche

1. Reaktive mesogene Benzodithiophene, bestehend aus einem zentralen mesogenen Kern, der eine oder mehrere Benzodithiophengruppen enthält und gegebenenfalls weitere ungesättigte organische Gruppen enthält, die zusammen mit den Benzodithiophengruppen ein konjugiertes System bilden, wobei der mesogene Kern, gegebenenfalls über eine Spacergruppe, mit einer oder mehreren reaktiven Gruppen verknüpft ist.

2. Reaktive mesogene Benzodithiophene nach Anspruch 1, **dadurch gekennzeichnet, dass** sie aus der Formel I
P-Sp-X-T-R I
ausgewählt sind, worin
P eine polymerisierbare oder reaktive Gruppe bedeutet,
Sp eine Spacergruppe oder eine Einfachbindung bedeutet,
X eine Verknüpfungsgruppe oder eine Einfachbindung bedeutet,
R H, Halogen, CN, NO₂, eine aliphatische, alicyclische oder aromatische Gruppe mit bis zu 40 C-Atomen, die gegebenenfalls ein oder mehrere Heteroatome enthält und worin ein oder mehrere Ringe anelliert sein können, oder P-Sp- bedeutet, und
T eine mesogene Gruppe enthaltend eine oder mehrere Benzodithiophengruppen bedeutet, die substituiert oder unsubstituiert sein können.

3. Reaktive mesogene Benzodithiophene nach Anspruch 2, **dadurch gekennzeichnet, dass** T aus der Formel II
-Z¹-(A¹-Z²)ₘ-(T¹-Z³)ₙ-(A²-Z⁴)ₒ- II
ausgewählt ist, worin
A¹ und A² unabhängig voneinander eine aromatische, hetero-aromatische oder alicyclische Gruppe mit bis zu 18 C-Atomen bedeuten, die unsubstituiert oder ein- oder mehrfach mit R¹ substituiert ist, und A¹ auch T¹ bedeuten kann,
Z¹ bis Z⁴ unabhängig voneinander -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, CY¹=CY²-, -C≡C-, -CH=CH-COO-, -OCO-CH=CH- oder eine Einfachbindung bedeuten,
Y¹ und Y² unabhängig voneinander H, F, Cl oder CN bedeuten,
T¹ eine Gruppe bedeutet, die aus 1 oder 2 Benzodithiopheneinheiten besteht, die gegebenenfalls durch R¹ substituiert sind,
R¹ H, Halogen, CN, NO₂, geradkettiges, verzweigtes oder cyclisches Alkyl mit 1 bis 20 C-Atomen bedeutet, das unsubstituiert oder ein- oder mehrfach durch F, Cl, Br, I oder CN substituiert ist und worin gegebenenfalls eine oder mehrere nicht benachbarte CH₂-Gruppen jeweils unabhängig voneinander durch -O-, -S-, -NH-, -NR⁰-, -SiR⁰R⁰⁰-, -CO-, -COO-, -OCO-, -OCO-O-, -S-CO-, -CO-S-, -CY¹=CY²- oder -C≡C- so ersetzt sind, dass O- und/oder S-Atome nicht direkt miteinander verknüpft sind, oder eine aromatische oder heteroaromatische Gruppe oder P-Sp-X bedeutet,
R⁰ und R⁰⁰ unabhängig voneinander H oder Alkyl mit 1 bis 12 C-Atomen bedeuten,
m und o unabhängig voneinander 0, 1, 2 oder 3 bedeuten und
n 1, 2 oder 3 bedeutet.

4. Reaktive mesogene Benzodithiophene nach Anspruch 3, **dadurch gekennzeichnet, dass** T¹ aus den folgenden Unterformeln ausgewählt ist 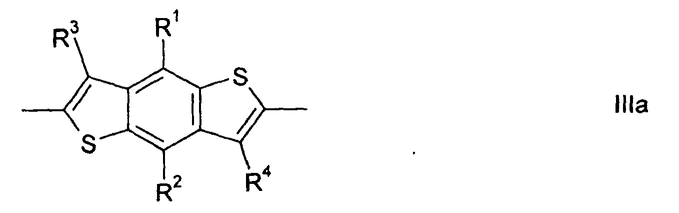 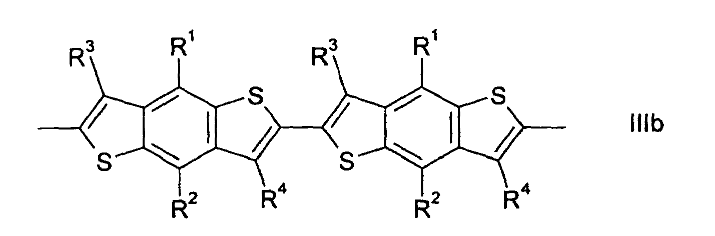 worin R¹ bis R⁴ unabhängig voneinander H, Halogen, CN, NO₂, geradkettiges, verzweigtes oder cyclisches Alkyl mit 1 bis 20 C-Atomen bedeuten, das unsubstituiert oder ein- oder mehrfach durch F, Cl, Br, I oder CN substituiert ist und worin gegebenenfalls eine oder mehrere nicht benachbarte CH₂-Gruppen jeweils unabhängig voneinander durch -O-, -S-, -NH-, -NR°-, -SiR⁰R⁰⁰-, -CO-, -COO-, -OCO-, -OCO-O-, -S-CO-, -CO-S-, -CY¹=CY²- oder -C=C- so ersetzt sind, dass O- und/oder S-Atome nicht direkt miteinander verknüpft sind, oder eine aromatische oder heteroaromatische Gruppe oder P-Sp-X bedeuten.

5. Reaktive mesogene Benzodithiophene nach Anspruch 3 und/oder 4, **dadurch gekennzeichnet, dass** A¹ und A² ausgewählt sind aus 1,4-Phenylen, 1,4-Cyclohexa-1,3-dien, 1,4-Cyclohexenylen, wobei zusätzlich eine oder mehrere CH-Gruppen gegebenenfalls durch N oder eine oder zwei nicht benachbarte CH₂-Gruppen gegebenenfalls durch O und/oder S ersetzt sind, Thiophen-2,5-diyl, Thienothiophen-2,5-diyl, Dithienothiophen-2,6-diyl, 1,4-Bicyclo-(2,2,2)-octylen, Naphthalin-2,6-diyl, Furan-2,5-diyl und Indan-2,5-diyl, wobei alle diese Gruppen unsubstituiert oder ein- oder mehrfach durch L substituiert sind, wobei L F, Cl, Br, CN, SCN, NO₂, SF₅ oder eine Alkyl-, Alkoxy-, Alkylcarbonyl- oder Alkoxycarbonylgruppe mit 1 bis 12 C-Atomen bedeutet, worin ein oder mehrere H-Atome gegebenenfalls durch F oder Cl ersetzt sind.

6. Reaktive mesogene Benzodithiophene nach mindestens einem der Ansprüche 3 bis 5, **dadurch gekennzeichnet, dass** sie aus den folgenden Formeln ausgewählt sind 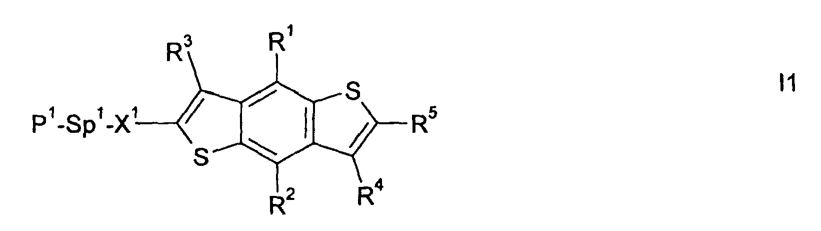 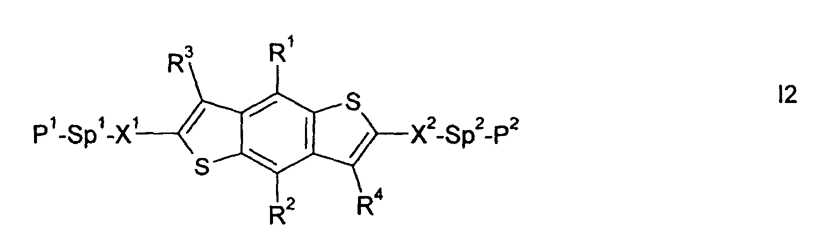 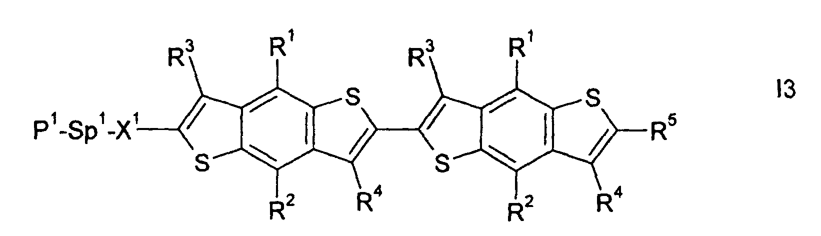 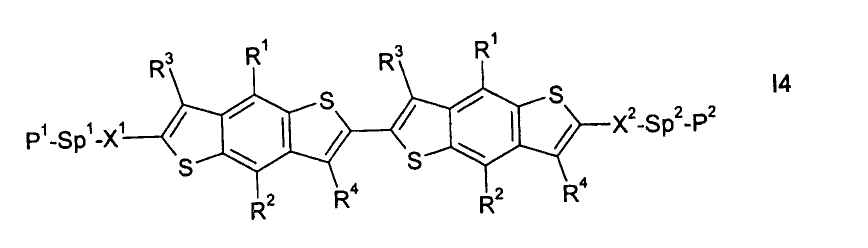 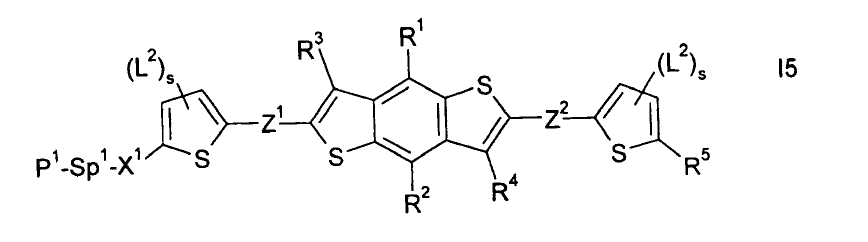 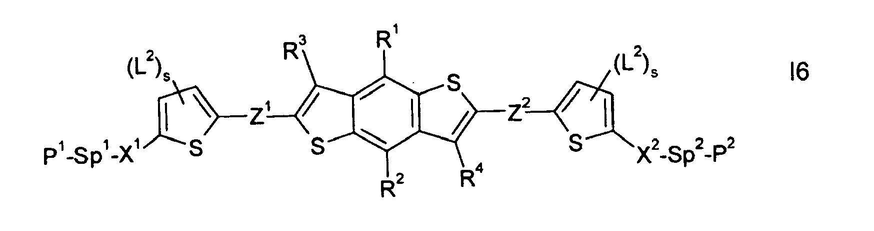 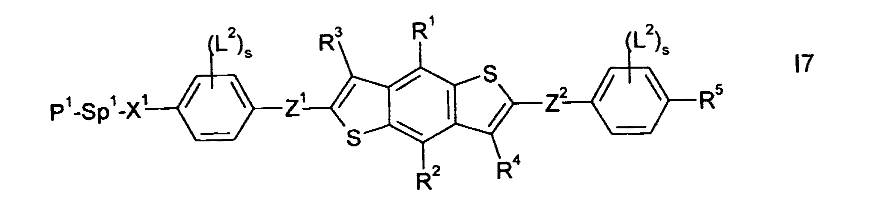 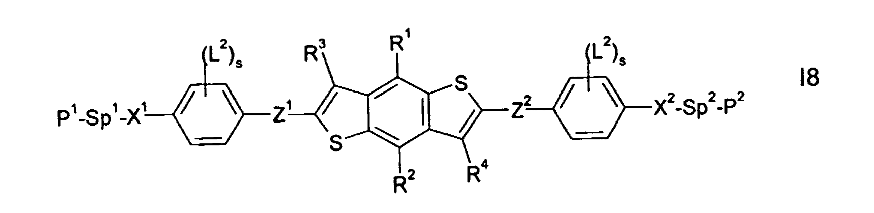 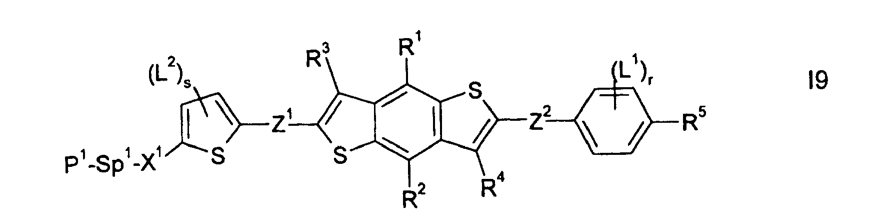 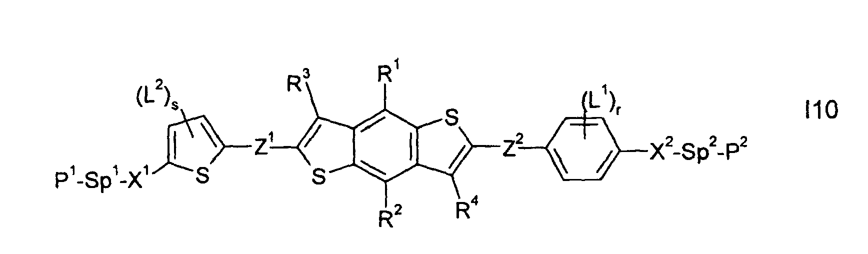 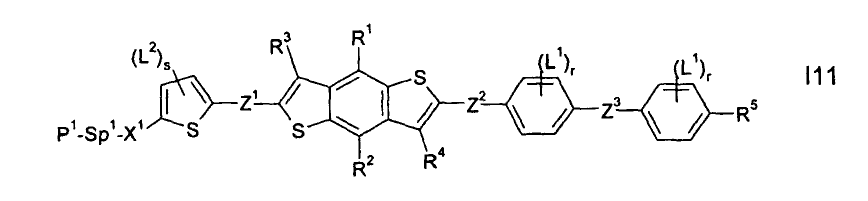 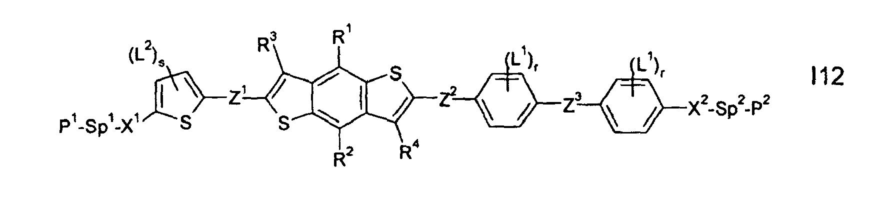 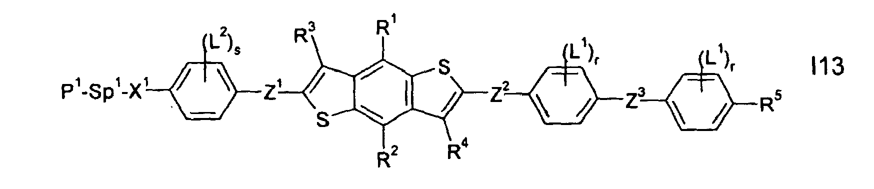 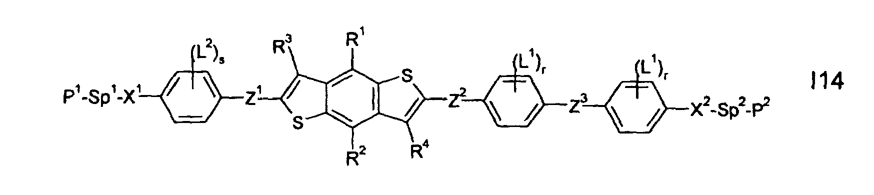 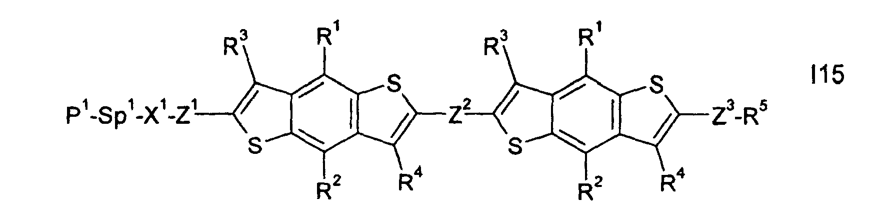 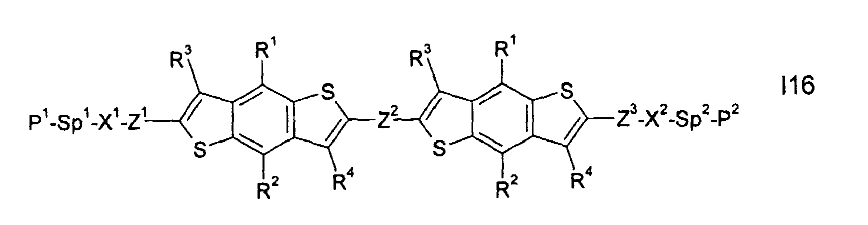 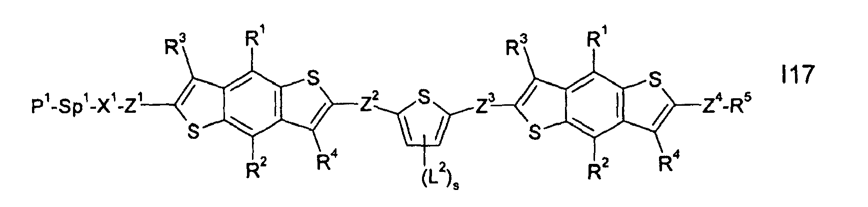 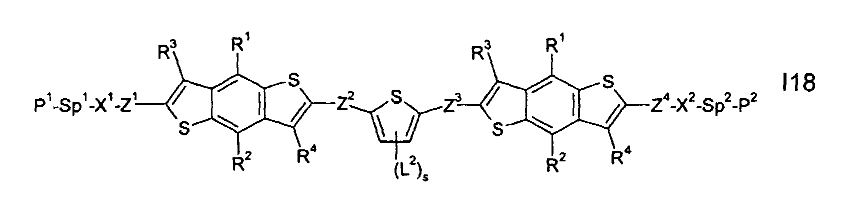 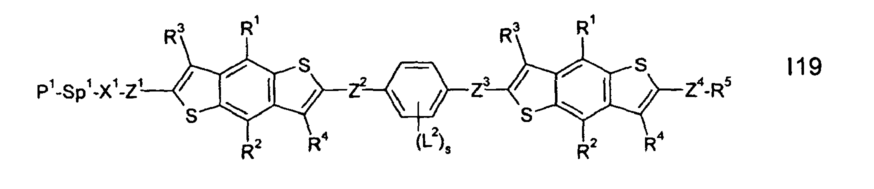 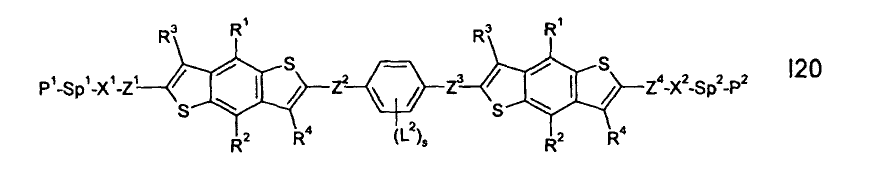 worin
P¹ und P² gleiche oder verschiedene Gruppen P wie in Anspruch 2 definiert bedeuten,
Sp¹ und Sp² gleiche oder verschiedene Gruppen Sp wie in Anspruch 2 definiert bedeuten,
X¹ und X² gleiche oder verschiedene Gruppen X wie in Anspruch 2 definiert bedeuten,
Z¹ bis Z⁴ wie in Formel II definiert sind und vorzugsweise -CH=CH-, -CH=CF-, -CF=CH-, CH=CCl-, -CCl=CH-, -CF=CF-, -CCl=CCl-, -C≡C- oder eine Einfachbindung bedeuten,
R¹ bis R⁴ unabhängig voneinander eine der Bedeutungen von R¹ in Formel II besitzen und vorzugsweise Halogen oder eine gegebenenfalls fluorierte Alkylgruppe mit 1 bis 15 C-Atomen bedeuten,
R⁵ eine der Bedeutungen von R¹ in Formel II besitzt und vorzugsweise Halogen oder eine gegebenenfalls fluorierte Alkylgruppe mit 1 bis 15 C-Atomen bedeutet,
L¹ eine der Bedeutungen von L wie in Anspruch 5 definiert besitzt und vorzugsweise F, Cl oder Alkyl oder Alkoxy mit 1 bis 3 C-Atomen bedeutet, das einfach, mehrfach oder perfluoriert ist,
L² eine der Bedeutungen von L wie in Anspruch 5 definiert besitzt und vorzugsweise Alkyl mit 1 bis 12 C-Atomen bedeutet, das einfach, mehrfach oder perfluoriert ist,
r 0, 1, 2, 3 oder 4 bedeutet und
s 0, 1 oder 2 bedeutet.

7. Reaktive mesogene Benzodithiophene nach mindestens einem der Ansprüche 1 bis 6, **dadurch gekennzeichnet, dass** die polymerisierbare oder reaktive Gruppe oder Gruppe P ausgewählt ist aus CH₂=CW¹-COO-, 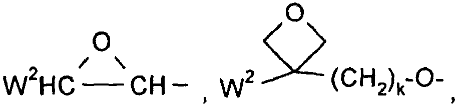 CH²=CW²(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- und W⁴W⁵W⁶Si-, wobei W¹ H, Cl, CN, Phenyl oder Alkyl mit 1 bis 5 C-Atomen, insbesondere H, Cl oder CH₃ bedeutet, W² und W³ unabhängig voneinander H oder Alkyl mit 1 bis 5 C-Atomen, insbesondere Methyl, Ethyl oder n-Propyl bedeuten, W⁴, W⁵ und W⁶ unabhängig voneinander Cl, Oxaalkyl oder Oxacarbonylalkyl mit 1 bis 5 C-Atomen bedeuten, Phe 1,4-Phenylen bedeutet und k₁ und k₂ unabhängig voneinander 0 oder 1 bedeuten.

8. Reaktive mesogene Benzodithiophene nach mindestens einem der Ansprüche 1 bis 7, **dadurch gekennzeichnet, dass** sie eine Flüssigkristallphase aufweisen.

9. Reaktive Flüssigkristallmischung enthaltend ein oder mehrere reaktive mesogene Benzodithiophene nach mindestens einem der Ansprüche 1 bis 8 und gegebenenfalls eine oder mehrere weitere reaktive Verbindungen, worin mindestens eines der Benzodithiophene und weiteren reaktiven Verbindungen mesogen oder flüssigkristallin ist.

10. Anisotrope Polymerfolie mit ladungstransportierenden Eigenschaften, erhältlich aus einer reaktiven Flüssigkristallmischung nach Anspruch 9, die in ihrer Flüssigkristallphase in makroskopisch uniforme Ausrichtung orientiert und zur Fixierung des ausgerichteten Zustandes polymerisiert oder vernetzt ist.

11. Seitenketten-Flüssigkristallpolymer erhalten durch Polymerisation einer oder mehrerer Verbindungen oder eines reaktiven Materials nach den Ansprüchen 1 bis 9 oder durch Aufpfropfen einer oder mehrerer Verbindungen oder eines reaktiven Materials nach den Ansprüchen 1 bis 9 in einer polymeranalogen Reaktion auf ein Polymerrückgrat, gegebenenfalls mit einem oder mehreren zusätzlichen mesogenen oder nicht mesogenen Comonomeren.

12. Verwendung der reaktiven mesogenen Benzodithiophene, polymerisierbaren Mischungen und Polymere nach mindestens einem der Ansprüche 1 bis 11 als Halbleiter oder Ladungstransportmaterialien, insbesondere in optischen, elektrooptischen oder elektronischen Vorrichtungen, wie beispielsweise Komponenten integrierter Schaltungen, Feldeffekttransistoren (FET) beispielsweise als Dünnfilmtransistoren in Flachbildschirm-Anwendungen oder für RFID-Tags (Radio Frequency Identification), oder in Halbleiterkomponenten für Anwendungen mit organischen Leuchtdioden (OLED) wie Elektrolumineszenzanzeigen oder Hintergrundbeleuchtungen von z.B. Flüssigkristallanzeigen, für photovoltaische oder Sensorvorrichtungen, als Elektrodenmaterialien in Batterien, als Photoleiter und für elektrophotographische Anwendungen wie elektrophotographische Aufzeichnung, oder als lichtmodulierende Komponenten für Flüssigkristallanzeigen, optische Folien oder andere optische oder elektrooptische Vorrichtungen.

13. Feldeffekttransistor, beispielsweise als Komponente integrierter Schaltungen, als Dünnfilmtransistor in Flachbildschirm-Anwendungen oder in einem RFID-Tag (Radio Frequency Identification), enthaltend ein oder mehrere reaktive mesogene Benzodithiophene, reaktive Mischungen oder Polymere nach mindestens einem der Ansprüche 1 bis 11.

14. Sicherheitsmarkierung oder -vorrichtung enthaltend ein oder mehrere reaktive mesogene Benzodithiophene, reaktive Mischungen oder Polymere nach mindestens einem der Ansprüche 1 bis 11 oder ein FET oder RFID-Tag nach Anspruch 13.

15. Reaktive mesogene Benzodithiophene, reaktive Mischungen oder Polymere nach mindestens einem der Ansprüche 1 bis 11, die oxidativ oder reduktiv dotiert sind, so dass sie leitende Ionenspezies bilden.

16. Ladungsinjektionsschicht, Planarisierungsschicht, Antistatikfolie oder leitendes Substrat oder Muster für Elektronikanwendungen oder Flachbildschirme, enthaltend ein oder mehrere reaktive mesogene Benzodithiophene, reaktive Mischungen oder Polymerfolien nach Anspruch 15.

## Revendications

1. Benzodithiophènes mésogènes réactifs, constitués par un noyau mésogène central qui comprend un ou plusieurs benzodithiophènes et qui comprend en option d'autres groupes organiques non saturés qui forment un système conjugué en association avec les groupes benzodithiophène, ledit noyau mésogène étant lié, en option via un groupe d'espaceur, à un ou plusieurs groupes réactifs.

2. Benzodithiophènes mésogènes réactifs selon la revendication 1, **caractérisés en ce qu'**ils sont choisis à partir de la formule I
P-Sp-X-T-R I
dans laquelle
P est un groupe polymérisable ou réactif,
Sp est un groupe d'espaceur ou une liaison simple,
X est un groupe de liaison ou une liaison simple,
R est H, halogène, CN, NO₂, un groupe aliphatique, alicyclique ou aromatique avec jusqu'à 40 atomes de C qui comprend en option un ou plusieurs hétéroatomes et où un ou plusieurs anneaux peuvent être fusionnés, ou P-Sp-X-, et
T est un groupe mésogène qui comprend un ou plusieurs groupes benzodithiophène qui sont substitués ou non substitués.

3. Benzodithiophènes mésogènes réactifs selon la revendication 2, **caractérisés en ce que** T est choisi à partir de la formule II
-Z¹-(A¹-Z²)ₘ-(T¹-Z³)ₙ-(A²-Z⁴)ₒ- II
dans laquelle
A¹ et A² sont, indépendamment l'un de l'autre, un groupe aromati-que, hétéroaromatique ou alicyclique avec jusqu'à 18 atomes de C qui est non substitué, mono- ou polysubstitué par R¹, et A¹ peut également représenter T¹,
Z¹ à Z⁴ sont, indépendamment les uns des autres, -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C-, -CH=CH-COO-, -OCO-CH=CH- ou une liaison simple,
Y¹ et Y² sont, indépendamment l'un de l'autre, H, F, Cl ou CN,
T¹ est un groupe constitué par une ou deux unités de benzodithiophène qui sont en option substituées par R¹,
R¹ est H, halogène, CN, NO₂, un alkyle en chaîne droite, ramifié ou cyclique avec de 1 à 20 atomes de C, qui est non substitué, mono- ou polysubstitué par F, Cl, Br, I ou CN et où un ou plusieurs groupes CH₂ non adjacents sont en option remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CY¹=CY²- ou -C≡C- de telle sorte que des atomes de O et/ou de S ne soient pas liés directement les uns aux autres, un groupe aromatique ou hétéroaromatique, ou P-Sp-X,
R⁰ et R⁰⁰ sont, indépendamment l'un de l'autre, H ou alkyle avec de 1 à 12 atomes de C,
m et o sont, indépendamment l'un de l'autre, 0,1, 2 ou 3, et
n est 1, 2 ou 3.

4. Benzodithiophènes mésogènes réactifs selon la revendication 3, **caractérisés en ce que** T¹ est choisi à partir des sous-formules qui suivent 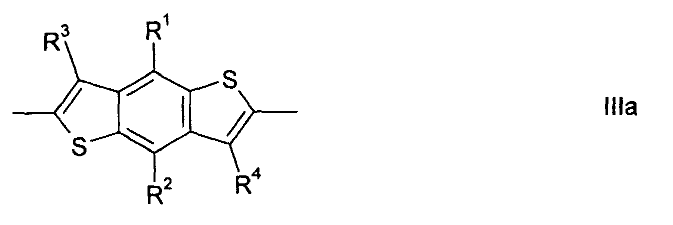 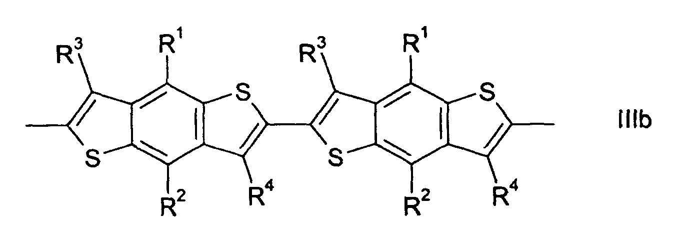 dans lesquelles R¹ à R⁴ sont, indépendamment les uns des autres, H, halogène, CN, NO₂, un alkyle en chaîne droite, ramifié ou cyclique avec de 1 à 20 atomes de C, qui est non substitué, mono- ou polysubstitué par F, CI, Br, I ou CN et dans lequel un ou plusieurs groupes CH₂ non adjacents sont en option remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CY¹=CY²- ou -C≡C- de telle sorte que des atomes de O et/ou de S ne soient pas liés directement les uns aux autres, un groupe aromatique ou hétéroaromatique, ou P-Sp-X.

5. Benzodithiophènes mésogènes réactifs selon la revendication 3 et/ou 4, **caractérisés en ce que** A¹ et A² sont choisis parmi 1,4-phénylène, 1,4-cyclohexa-1,3-diène, 1,4-cyclohexénylène où, en plus, un ou plusieurs groupes CH sont en option remplacés par N ou un ou deux groupes CH₂ non adjacents sont en option remplacés par O et/ou S, thiophène-2,5-diyle, thiénothiophène-2,5-diyle, dithiénothiophène-2,6-diyle, 1,4-bicyclo-(2,2,2)-octylène, naphthalène-2,6-diyle, furane-2,5-diyle et indane-2,5-diyle, tous ces groupes étant non substitués, mono- ou polysubstitués par L, L étant F, Cl, Br, CN, SCN, NO₂, SF₅ ou un groupe alkyle, alkoxy, alkylcarbonyle ou alkoxycarbonyle avec de 1 à 12 atomes de C, où un ou plusieurs atomes de H sont en option remplacés par F ou Cl.

6. Benzodithiophènes mésogènes réactifs selon au moins l'une des revendications 3 à 5, **caractérisés en ce qu'**ils sont choisis à partir des formules qui suivent 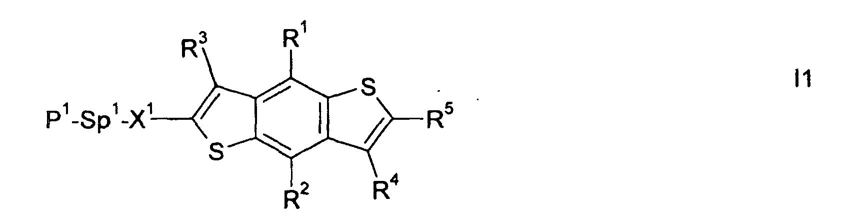 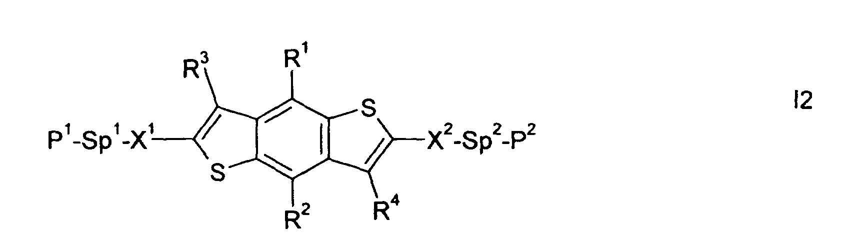 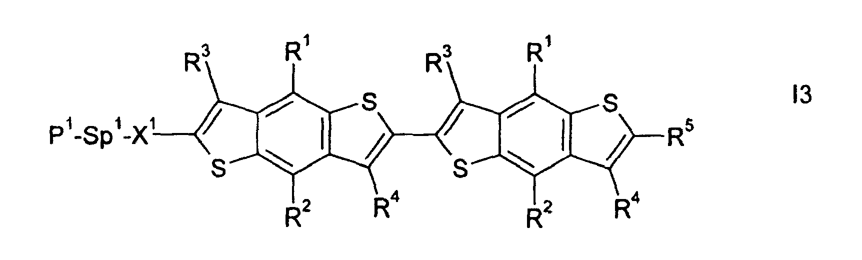 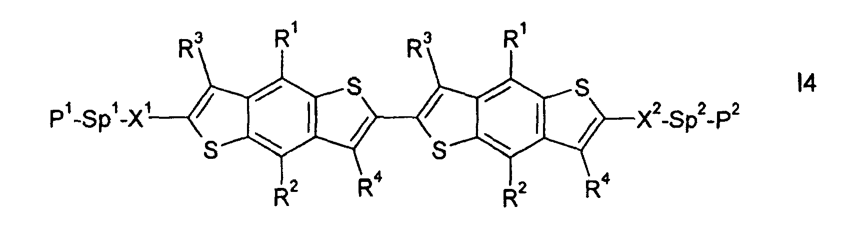 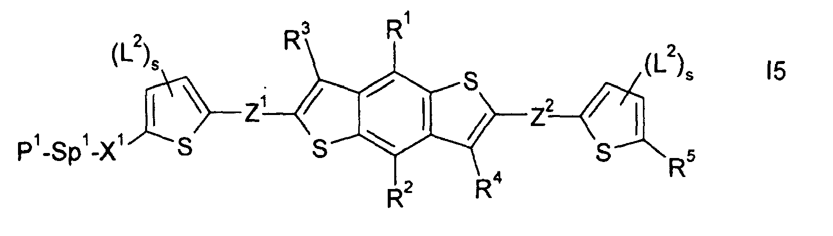 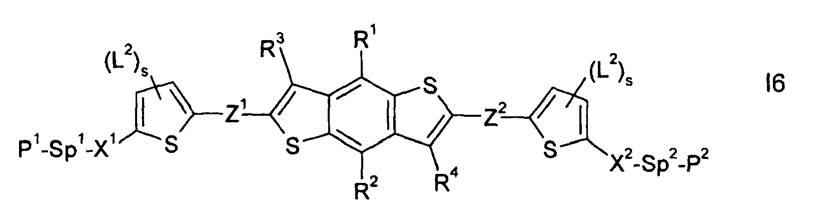 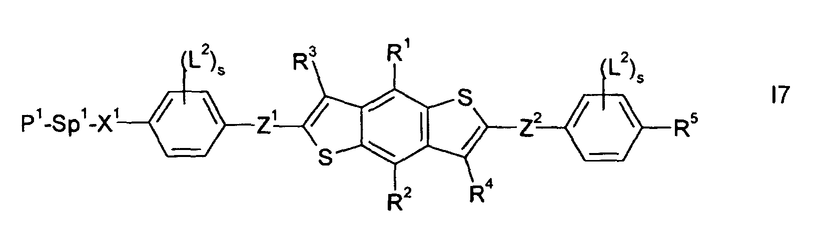 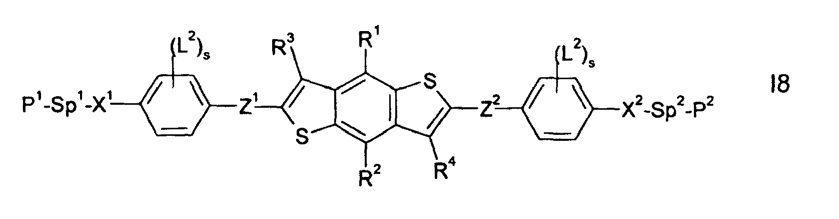 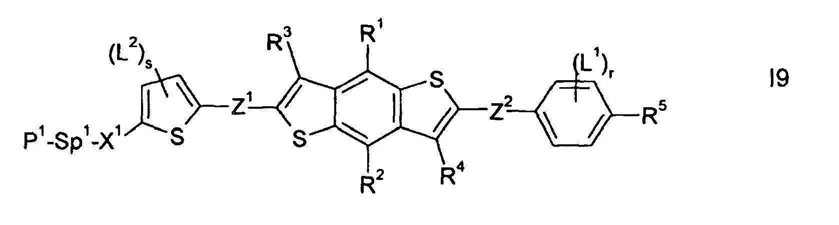 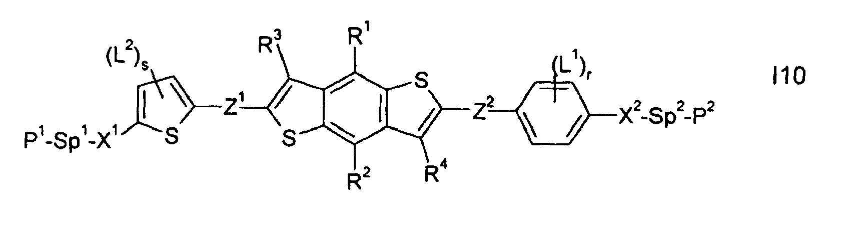 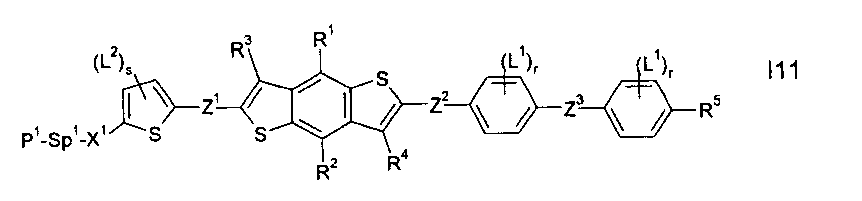 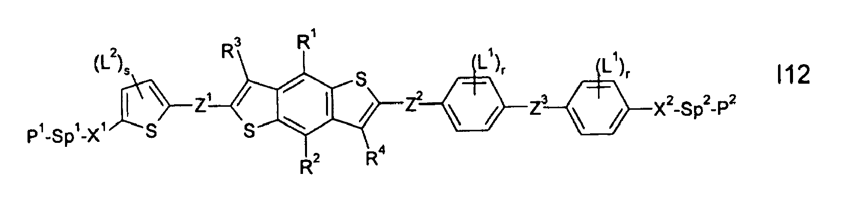 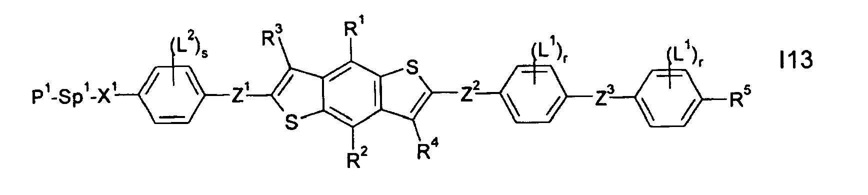 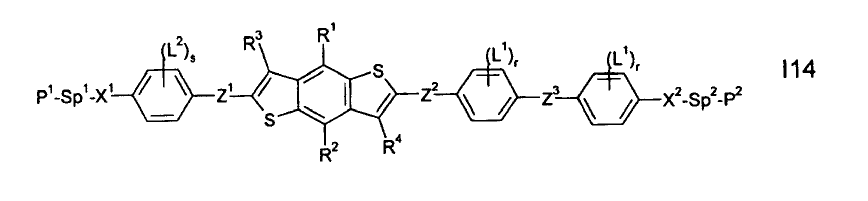 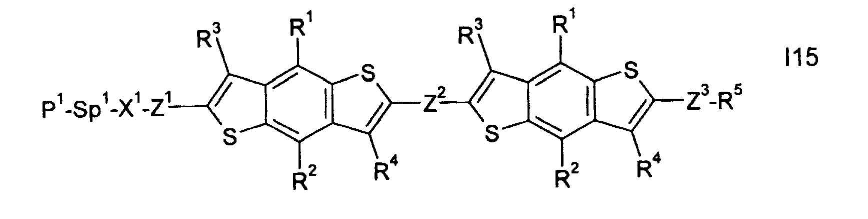 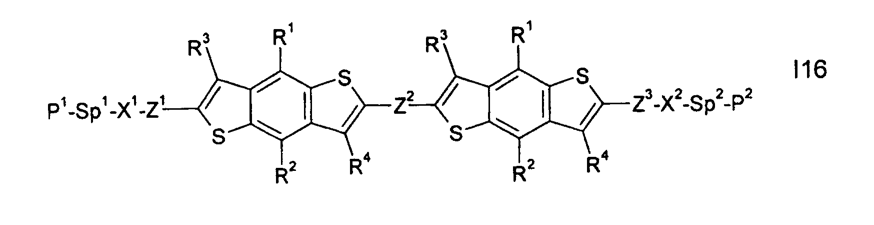 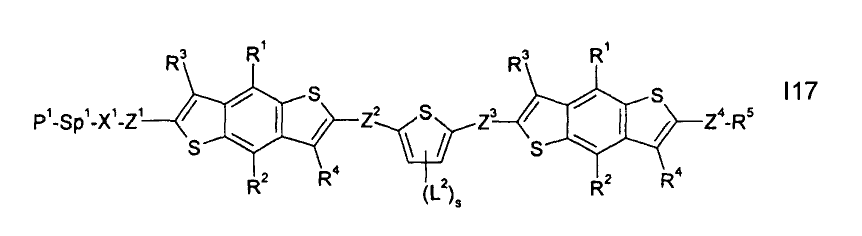 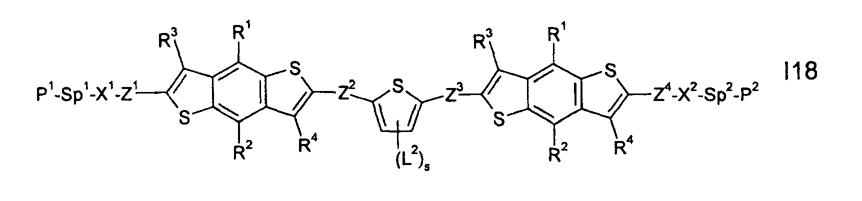 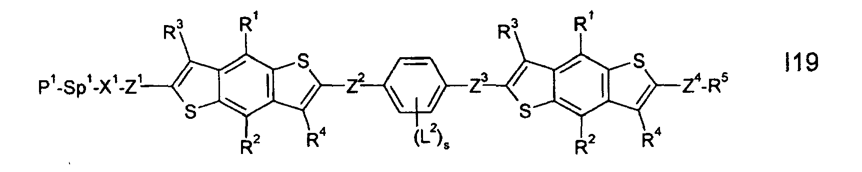 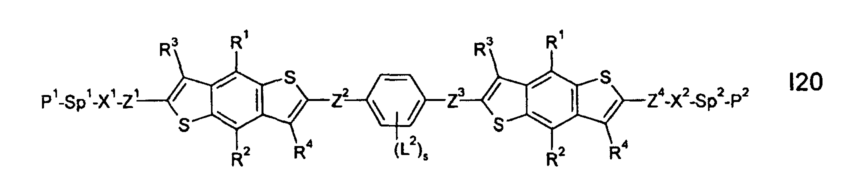 dans lesquelles
P¹ et P² sont des groupes P identiques ou différents tels que définis selon la revendication 2,
Sp¹ et Sp² sont des groupes Sp identiques ou différents tels que définis selon la revendication 2,
X¹ et X² sont des groupes X identiques ou différents tels que définis selon la revendication 2,
Z¹ à Z⁴ sont tels que définis selon la formule II et sont de préférence -CH=CH-, -CH=CF-, -CF=CH-, CH=CCl-, -CCl=CH-, -CF=CF-, -CCl=CCl-, -C=C- ou une liaison simple,
R¹ à R⁴ présentent, indépendamment les uns des autres, l'une des significations de R¹ dans la formule II et sont de préférence halogène ou un groupe alkyle en option fluoré avec de 1 à 15 atomes de C,
R⁵ présente l'une des significations de R¹ dans la formule II et est de préférence halogène ou un groupe alkyle en option fluoré avec de 1 à 15 atomes de C,
L¹ présente l'une des significations de L telles que définies selon la revendication 5 et est de préférence F, Cl ou alkyle ou alkoxy avec de 1 à 3 atomes de C qui est en option mono-, poly- ou perfluoré,
L² présente l'une des significations de L telles que définies selon la revendication 5 et est de préférence alkyle avec de 1 à 12 atomes de C qui est en option mono-, poly- ou perfluoré,
r est 0, 1, 2, 3 ou 4, et
s est 0, 1 ou 2.

7. Benzodithiophènes mésogènes réactifs selon au moins l'une des revendications 1 à 6, **caractérisés en ce que** le groupe polymérisable ou réactif ou le groupe P est choisi parmi CH₂=CW¹-COO-, 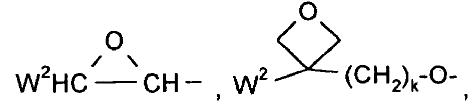 CH₂=CW²-(O)ₖ₁-, CH₃₋CH=CH-O-, (CH₂=CH)₂CH-OCO-, (CH₂=CH-CH₂)₂CH-OCO-, (CH₂=CH)₂-CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁₋Phe-(O)ₖ₂-, Phe-CH=CH-, HOOC-, OCN- et W⁴W⁵W⁶Si-, W¹ étant H, CI, CN, phényle ou alkyle avec de 1 à 5 atomes de C, en particulier H, Cl ou CH₃, W² et W³ étant, indépendamment l'un de l'autre, H ou alkyle avec de 1 à 5 atomes de C, en particulier méthyle, éthyle ou n-propyle, W⁴, W⁵ et W⁶ étant, indépendamment les uns des autres, Cl, oxaalkyle ou oxacarbonylalkyle avec de 1 à 5 atomes de C, Ph étant 1,4-phénylène et k₁ et k₂ étant, indépendamment l'un de l'autre, 0 ou 1.

8. Benzodithiophènes mésogènes réactifs selon au moins l'une des revendications 1 à 7, **caractérisés en ce qu'**ils présentent une phase de cristal liquide.

9. Mélange de cristal liquide réactif comprenant un ou plusieurs benzodithiophènes mésogènes réactifs selon au moins l'une des revendications 1 à 8 et en option, un ou plusieurs autres composés réactifs, où au moins l'un desdits benzodithiophènes et des autres composés réactifs est mésogène ou cristallin liquide.

10. Film en polymère anisotrope présentant des propriétés de transport de charge pouvant être obtenu à partir d'un mélange de cristal liquide réactif selon la revendication 9 qui est aligné selon sa phase de cristal liquide selon une orientation uniforme macroscopiquement et qui est polymérisé ou réticulé pour fixer l'état orienté.

11. Polymère de cristal liquide en chaîne latérale obtenu par polymérisation d'un ou de plusieurs composés ou d'un matériau réactif selon les revendications 1 à 9 ou par greffage d'un ou de plusieurs composés ou d'un matériau réactif selon les revendications 1 à 9 sur une ossature de polymère lors d'une réaction polymère analogue, en option avec un ou plusieurs comonomères mésogènes ou non mésogènes additionnels.

12. Utilisation des benzodithiophènes mésogènes réactifs, des mélanges polymérisables et des polymères selon au moins l'une des revendications 1 à 11 en tant que semiconducteurs ou que matériaux de transport de charge, en particulier dans des dispositifs optiques, électro-optiques ou électroniques, tels que par exemple des composants de circuit intégré, des transistors à effet de champ (FET) par exemple en tant que transistors à film mince dans des applications d'affichage à écran plat ou pour des étiquettes d'identification haute fréquence (RFID), ou dans des composants de semiconduction pour des applications de diodes émettrices de lumière organiques (DELO) en tant qu'affichages électroluminescents ou en tant qu'éclairages arrière par exemple d'affichages à cristaux liquides, pour des dispositifs photovoltaïques ou de capteur, en tant que matériaux d'électrode dans des accumulateurs, en tant que photoconducteurs et pour des applications électrophotographiques telles qu'un enregistrement électrophotographique ou en tant que composants de modulation de lumière pour des affichages à cristaux liquides, des films optiques ou d'autres dispositifs optiques ou électro-optiques.

13. Transistor à effet de champ, par exemple en tant que composant d'un circuit intégré, en tant que transistor à film mince dans des applications d'affichage à écran plat ou au niveau d'une étiquette d'identification haute fréquence (RFID), comprenant un ou plusieurs benzodithiophènes mésogènes réactifs, mélanges de réaction ou polymères selon au moins l'une des revendications 1 à 11.

14. Marquage ou dispositif de sécurité comprenant un ou plusieurs benzodithiophènes mésogènes réactifs, mélanges réactifs ou polymères selon au moins l'une des revendications 1 à 11, ou un FET ou une étiquette RFID selon la revendication 13.

15. Benzodithiophènes mésogènes réactifs, mélanges réactifs ou polymères selon au moins l'une des revendications 1 à 11, qui sont dopés par oxydation ou réduction pour former des espèces ioniques conductrices.

16. Couche d'injection de charge, couche de planarisation, film antistatique ou substrat ou motif de conduction pour des applications électroniques ou des affichages à écran plat, comprenant un ou plusieurs benzodithiophènes mésogènes réactifs, mélanges réactifs ou films en polymère selon la revendication 15.
